(19) Europäisches Patentamt
European Patent Office
Office européen des brevets

(11) EP 2 424 994 B1

(12) **EUROPEAN PATENT SPECIFICATION**

(45) Date of publication and mention of the grant of the patent:
**12.08.2015 Bulletin 2015/33**

(21) Application number: **10713984.2**

(22) Date of filing: **19.04.2010**

(51) Int Cl.:
***C12N 15/82*** *(2006.01)* ***C07K 14/415*** *(2006.01)*
***A01H 5/00*** *(2006.01)* ***A01H 5/10*** *(2006.01)*

(86) International application number:
**PCT/EP2010/055099**

(87) International publication number:
**WO 2010/124953 (04.11.2010 Gazette 2010/44)**

(54) **PLANTS HAVING ENHANCED YIELD-RELATED TRAITS AND A METHOD FOR MAKING THE SAME**

PFLANZEN MIT EIGENSCHAFTEN IN VERBINDUNG MIT VERBESSERTEM ERTRAG SOWIE VERFAHREN ZU DEREN HERSTELLUNG

PLANTES AYANT DES CARACTÈRES LIÉS AU RENDEMENT AMPLIFIÉS ET LEUR PROCÉDÉ DE FABRICATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **08.07.2009 US 223712 P**
**26.05.2009 US 180948 P**
**07.07.2009 EP 09164832**
**29.04.2009 EP 09159059**

(43) Date of publication of application:
**07.03.2012 Bulletin 2012/10**

(60) Divisional application:
**15175599.8**

(73) Proprietors:
- **BASF Plant Science Company GmbH**
**67056 Ludwigshafen (DE)**
- **Crop Functional Genomics Center**
**Gwanak-Gu**
**Seoul 151-921 (KR)**

(72) Inventors:
- **KIM, Ju Kon**
**Sungnam 463-846 (KR)**
- **CHOI, Yang Do**
**Seoul 137-909 (KR)**
- **JEONG, Jin Seo**
**Yongin 449-706 (KR)**
- **REUZEAU, Christophe**
**F-24350 Tocan Saint Apre (FR)**

(74) Representative: **Mistry, Meeta et al**
**Greaves Brewster LLP**
**Copa House**
**Station Road**
**Cheddar, BS27 3AH (GB)**

(56) References cited:
**WO-A1-2009/034188** **WO-A2-03/008540**
**WO-A2-2007/117693** **WO-A2-2009/016232**
**US-A1- 2004 123 343**

- **PATER DE B S ET AL: "THE PROMOTER OF THE RICE GENE GOS2 IS ACTIVE IN VARIOUS DIFFERENT MONOCOT TISSUES AND BINDS RICE NUCLEAR FACTOR ASF-1", THE PLANT JOURNAL, BLACKWELL SCIENTIFIC PUBLICATIONS, OXFORD, GB, vol. 2, no. 6, 1 January 1992 (1992-01-01) , pages 837-844, XP000907326, ISSN: 0960-7412, DOI: 10.1111/J. 1365-313X.1992.00837.X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 424 994 B1

SEQUENCE LISTING

<110> BASF Plant Science Company GmbH
Crop Functional Genomics Center

<120> Plants having enhanced yield-related traits and a method for making the same

<130> PF62069_PCT

<160> 403

<170> PatentIn version 3.3

<210> 1
<211> 1008
<212> DNA
<213> Oryza sativa

<400> 1

```
atggaagaag ggtccaacaa aagagaagga cttcctcctc aactcctaga tctcatccca      60
gatgagaagg aatggaagct gagagaagca ttaggtctag ggagatcaag aaacgcaggc     120
ttcgacggtg aagaagacaa gaagctagac ctaaagcttg gtcttcctgg ttttatagaa     180
gatgatgagg cagaaacctt aagagattac aggctacagc aagagagccc atctctctcc     240
cttagtttct ttccaaagca ctccaagacc accagcagca ctactacgac aactggagca     300
aagaggggat tcatagacac agttgaggac aaaacagaag gttataacga ccagaagcag     360
caggcaaggg caggatgtgg gaaggaactt gcagtggagg aaatgatagc agccgtgagt     420
gagaggaaga aaggatgctg ccccccacca ccaccaccac atggtgctcc tgctacacct     480
gcgcgcaaca gaccacagac ccagggaaga ggagctgcag caccagtggt tggttggcct     540
ccaattcgat ccttcaggag aaaccttgct agtagtagtt catccaaaca ttcccctgaa     600
ccacaaaatg ataatgccaa tgcaaaggtg acgctcacct gcaagaaaaa ccctcttgta     660
aaaatcaaca tggatgggat ccctattgga aggaaaatag accttgcagc ctataacagc     720
tatgatgggc tatcgtcagc tgtcaaacaa ctcttccatg ggtttcttca agctcaaaag     780
gaccaaacta atgcgcaaat tgcacagcaa ggtgcagatg acaaaatatt ttatcaattg     840
ttggatgggt ctggtgaata tactctagtt tatgaagaca gtgaaggaga caggatgctg     900
gttggggacg tgccatggaa agtatttgtc tcaactgcta aaggctgag ggttctaagg      960
agttcagagc tttcccatac cctgattgga gctactgcta gggtctag                 1008
```

<210> 2
<211> 335
<212> PRT
<213> Oryza sativa

<400> 2

```
Met Glu Glu Gly Ser Asn Lys Arg Glu Gly Leu Pro Pro Gln Leu Leu
1               5                   10                  15
```

```
Asp Leu Ile Pro Asp Glu Lys Glu Trp Lys Leu Arg Glu Ala Leu Gly
            20                  25                  30

Leu Gly Arg Ser Arg Asn Ala Gly Phe Asp Gly Glu Glu Asp Lys Lys
        35                  40                  45

Leu Asp Leu Lys Leu Gly Leu Pro Gly Phe Ile Glu Asp Asp Glu Ala
    50                  55                  60

Glu Thr Leu Arg Asp Tyr Arg Leu Gln Gln Glu Ser Pro Ser Leu Ser
65                  70                  75                  80

Leu Ser Phe Phe Pro Lys His Ser Lys Thr Thr Ser Ser Thr Thr Thr
                85                  90                  95

Thr Thr Gly Ala Lys Arg Gly Phe Ile Asp Thr Val Glu Asp Lys Thr
            100                 105                 110

Glu Gly Tyr Asn Asp Gln Lys Gln Gln Ala Arg Ala Gly Cys Gly Lys
        115                 120                 125

Glu Leu Ala Val Glu Glu Met Ile Ala Ala Val Ser Glu Arg Lys Lys
    130                 135                 140

Gly Cys Cys Pro Pro Pro Pro Pro Pro His Gly Ala Pro Ala Thr Pro
145                 150                 155                 160

Ala Arg Asn Arg Pro Gln Thr Gln Gly Arg Gly Ala Ala Ala Pro Val
                165                 170                 175

Val Gly Trp Pro Pro Ile Arg Ser Phe Arg Arg Asn Leu Ala Ser Ser
            180                 185                 190

Ser Ser Ser Lys His Ser Pro Glu Pro Gln Asn Asp Asn Ala Asn Ala
        195                 200                 205

Lys Val Thr Leu Thr Cys Lys Lys Asn Pro Leu Val Lys Ile Asn Met
    210                 215                 220

Asp Gly Ile Pro Ile Gly Arg Lys Ile Asp Leu Ala Ala Tyr Asn Ser
225                 230                 235                 240

Tyr Asp Gly Leu Ser Ser Ala Val Lys Gln Leu Phe His Gly Phe Leu
                245                 250                 255

Gln Ala Gln Lys Asp Gln Thr Asn Ala Gln Ile Ala Gln Gln Gly Ala
            260                 265                 270
```

Asp Asp Lys Ile Phe Tyr Gln Leu Leu Asp Gly Ser Gly Glu Tyr Thr
275 280 285

Leu Val Tyr Glu Asp Ser Glu Gly Asp Arg Met Leu Val Gly Asp Val
290 295 300

Pro Trp Lys Val Phe Val Ser Thr Ala Lys Arg Leu Arg Val Leu Arg
305 310 315 320

Ser Ser Glu Leu Ser His Thr Leu Ile Gly Ala Thr Ala Arg Val
325 330 335

<210> 3
<211> 984
<212> DNA
<213> Oryza sativa

<400> 3

atggaagaag aattcaagga caaaggcctc cctcccacgc tgctacacct catccctgac 60

gggagagaat ggaaggtgaa agaagcagat ggagaaggat caaggaacac aaacttagat 120

gccgatgagg ataaggagct agagcttaag ctcggcctcc ctggtgtcca acaggaagag 180

agagcagctg attcaagaga gaagatacag cagcaacaac gagagagttc ctcagaacca 240

tccatcggtt gcttccctac acattcaaag cctaccacca gcatcggcac aactggggca 300

aagagaggat tttttgcgat tgttggggcc acactagaag gttataatca gagccaccgg 360

gacacagaag aatgtgggaa ggagctaacg ttaggagatg aaaatatggc aggtgagagg 420

aagaaaggat gctgtccctc accaccatgc tcagctgcag cgcacagcag caacccccag 480

gggagaggtg ctattcctcc agtagtcggt tggcctccaa tccggtcctt caggagaaac 540

ctcacaaatg gcagttcatt taaacagtcc cctgaacgac aaaatgacga agctgatgac 600

aaggcaaagc caatctgcaa gaagagacct cttgtcaaaa tcaacatgga tgggatcccc 660

attggaagga aagtagacct tcaaatatac gacagctacc agaagctgtc atctgctgtt 720

gaagagttgt tccgtggttt tcttgaagct caaaaagatc tatcttgtgc tgaaagtgga 780

gagcaagggg cagaagacaa aatattttca gggttgttgg atggaactgg tgtatacact 840

ttagtatatg aagacaatga tggagacaga atgttagctg gggacatacc atggaaggta 900

tttgtttcga ctgttaagag gttgagggtt atgaggaggt cagaacttcc ccatgacatg 960

attggagctg accctgtgaa gtag 984

<210> 4
<211> 327
<212> PRT

<213> Oryza sativa

<400> 4

**Met Glu Glu Glu Phe Lys Asp Lys Gly Leu Pro Pro Thr Leu Leu His**

```
1               5                   10                  15

Leu Ile Pro Asp Gly Arg Glu Trp Lys Val Lys Glu Ala Asp Gly Glu
            20                  25                  30

Gly Ser Arg Asn Thr Asn Leu Asp Ala Asp Glu Asp Lys Glu Leu Glu
        35                  40                  45

Leu Lys Leu Gly Leu Pro Gly Val Gln Gln Glu Glu Arg Ala Ala Asp
    50                  55                  60

Ser Arg Glu Lys Ile Gln Gln Gln Gln Arg Glu Ser Ser Ser Glu Pro
65                  70                  75                  80

Ser Ile Gly Cys Phe Pro Thr His Ser Lys Pro Thr Thr Ser Ile Gly
                85                  90                  95

Thr Thr Gly Ala Lys Arg Gly Phe Phe Ala Ile Val Gly Ala Thr Leu
            100                 105                 110

Glu Gly Tyr Asn Gln Ser His Arg Asp Thr Glu Glu Cys Gly Lys Glu
        115                 120                 125

Leu Thr Leu Gly Asp Glu Asn Met Ala Gly Glu Arg Lys Lys Gly Cys
    130                 135                 140

Cys Pro Ser Pro Pro Cys Ser Ala Ala Ala His Ser Ser Asn Pro Gln
145                 150                 155                 160

Gly Arg Gly Ala Ile Pro Pro Val Val Gly Trp Pro Pro Ile Arg Ser
                165                 170                 175

Phe Arg Arg Asn Leu Thr Asn Gly Ser Ser Phe Lys Gln Ser Pro Glu
            180                 185                 190

Arg Gln Asn Asp Glu Ala Asp Asp Lys Ala Lys Pro Ile Cys Lys Lys
        195                 200                 205

Arg Pro Leu Val Lys Ile Asn Met Asp Gly Ile Pro Ile Gly Arg Lys
    210                 215                 220

Val Asp Leu Gln Ile Tyr Asp Ser Tyr Gln Lys Leu Ser Ser Ala Val
225                 230                 235                 240

Glu Glu Leu Phe Arg Gly Phe Leu Glu Ala Gln Lys Asp Leu Ser Cys
                245                 250                 255

Ala Glu Ser Gly Glu Gln Gly Ala Glu Asp Lys Ile Phe Ser Gly Leu
            260                 265                 270
```

Leu Asp Gly Thr Gly Val Tyr Thr Leu Val Tyr Glu Asp Asn Asp Gly
275 280 285

Asp Arg Met Leu Ala Gly Asp Ile Pro Trp Lys Val Phe Val Ser Thr
290 295 300

Val Lys Arg Leu Arg Val Met Arg Arg Ser Glu Leu Pro His Asp Met
305 310 315 320

Ile Gly Ala Asp Pro Val Lys
325

<210> 5
<211> 588
<212> DNA
<213> Zea mays

<400> 5

atggcaggtg agacgaagaa agggtgctgc tgtcccccat cgtcgtcgca tgactcggat 60

gccgggcctg ctgtgcacag aggtgatgtt cttccggtgg ttggttggcc tccggtacgg 120

tccttcagga gaaacctggc aaatgccagc tcgtctaagc aatccctcga gcagcaacaa 180

caaaacgatg atgaagcctc ctgtgacaag gcgaagcaga cctgcaagag aagcccgctc 240

atcaaaataa acatggacgg catccccatt gggaggaaaa taaacctctc agcgtacgac 300

agctaccaga agctttcgtc tgccgtccaa gatttgttct gtggcttcct tgatgcccag 360

aaggatgaat ctagagggcg aggagcagaa gagaaaatgt tctcagggtt gttggatgga 420

actggtgagt acactttagt ttgtgaagac agtgaaggag gcaggacact agttgggcac 480

ctaccatgga atgtatttgt ttctaccgct aagaggctga gggtcatgga aagctctgaa 540

cttcctcatg gactgattaa aactgcctcc gagagggctg atgactga 588

<210> 6
<211> 195
<212> PRT
<213> Zea mays

<400> 6

```
Met Ala Gly Glu Thr Lys Lys Gly Cys Cys Cys Pro Pro Ser Ser Ser
1               5                   10                  15

His Asp Ser Asp Ala Gly Pro Ala Val His Arg Gly Asp Val Leu Pro
            20                  25                  30

Val Val Gly Trp Pro Pro Val Arg Ser Phe Arg Arg Asn Leu Ala Asn
        35                  40                  45

Ala Ser Ser Ser Lys Gln Ser Leu Glu Gln Gln Gln Gln Asn Asp Asp

    50                  55                  60

Glu Ala Ser Cys Asp Lys Ala Lys Gln Thr Cys Lys Arg Ser Pro Leu
65                  70                  75                  80

Ile Lys Ile Asn Met Asp Gly Ile Pro Ile Gly Arg Lys Ile Asn Leu
                85                  90                  95

Ser Ala Tyr Asp Ser Tyr Gln Lys Leu Ser Ser Ala Val Gln Asp Leu
            100                 105                 110

Phe Cys Gly Phe Leu Asp Ala Gln Lys Asp Glu Ser Arg Gly Arg Gly
        115                 120                 125

Ala Glu Glu Lys Met Phe Ser Gly Leu Leu Asp Gly Thr Gly Glu Tyr
    130                 135                 140

Thr Leu Val Cys Glu Asp Ser Glu Gly Gly Arg Thr Leu Val Gly His
145                 150                 155                 160

Leu Pro Trp Asn Val Phe Val Ser Thr Ala Lys Arg Leu Arg Val Met
                165                 170                 175

Glu Ser Ser Glu Leu Pro His Gly Leu Ile Lys Thr Ala Ser Glu Arg
            180                 185                 190

Ala Asp Asp
        195
```

<210> 7
<211> 1074
<212> DNA
<213> Zea mays

<400> 7

atgggagact ccggaggaag aagaggacca cctcttcctc ggctgctaga tctcatcccg 60
gacgggaaag aacggaaggg gagggaagcg caagacgcgg gaaggtcgaa gaacagcggc 120
ggcttcgggg gcgaggagga cgcgaagctg gagctcaagc tcgggcctcc gggtctcact 180
ctcgtagaag aaggaacgac agctaccgtg ccaagagatg gaggcgtact acagcgagag 240
acgacgacca ccccgaccct ctcgctcggg tgcttcccac gtaacccctc caagcctgcg 300
gtagacgcag ctacagctgg gacgaagagg gggttcttcg acaccgccgt cgaggccaag 360
acagaaggcc gcgacgagcg gatggagcag caggccggag ctggatgtgg gaacgagcta 420
gcactggacg agaagacggc agccgcggcc gcgagtgaga gacagaaagg gtcctgctgc 480
ccgccgacgc cacagcacgc ccctcctgct gcgaccgtgc acagcggagc acacgtcctg 540
cagctgggaa gaaggccttc ggctccggtt gtcggttggc cccggtcag atccttcagg 600
agaaaccttg ctcatcatca tcatggaagc tcctccaaac aacccaccga gccacagaac 660
agcgaagcta gcaggaagga gaagcctgcc tgcaagaaga accctctcgt aaaaatcaac 720
atggacggga tccccatcgg aaggaaagta gaccttgcag cctacgacag ctacgagagg 780
ctatcactgg gcgtcaaaga gcttttccac ggttttcttc aagctcaaaa ggatatgtct 840
ccaactgctg gaaagatatt ctctcaattg ttggatggat ctggcgaata taccctggtc 900
tacgaagaca acgaaggaga caggatgctg gtaggggacg tgccgtggaa tgtgtttgtg 960
tcaactgcta aaaggctgag ggttttgagg agctcggagc ttgccaaagg tttggtaggc 1020
acaaggccac ctcagattgc ggtagctccg agaagaggac caccagattg ctga 1074

<210> 8
<211> 357
<212> PRT
<213> Zea mays

<400> 8

```
Met Gly Asp Ser Gly Gly Arg Arg Gly Pro Pro Leu Pro Arg Leu Leu
1               5                   10                  15

Asp Leu Ile Pro Asp Gly Lys Glu Arg Lys Gly Arg Glu Ala Gln Asp
            20                  25                  30

Ala Gly Arg Ser Lys Asn Ser Gly Gly Phe Gly Gly Glu Glu Asp Ala
        35                  40                  45

Lys Leu Glu Leu Lys Leu Gly Pro Pro Gly Leu Thr Leu Val Glu Glu
    50                  55                  60

Gly Thr Thr Ala Thr Val Pro Arg Asp Gly Gly Val Leu Gln Arg Glu
65                  70                  75                  80

Thr Thr Thr Thr Pro Thr Leu Ser Leu Gly Cys Phe Pro Arg Asn Pro
                85                  90                  95

Ser Lys Pro Ala Val Asp Ala Ala Thr Ala Gly Thr Lys Arg Gly Phe
            100                 105                 110

Phe Asp Thr Ala Val Glu Ala Lys Thr Glu Gly Arg Asp Glu Arg Met
        115                 120                 125

Glu Gln Gln Ala Gly Ala Gly Cys Gly Asn Glu Leu Ala Leu Asp Glu
    130                 135                 140

Lys Thr Ala Ala Ala Ala Ala Ser Glu Arg Gln Lys Gly Ser Cys Cys
145                 150                 155                 160

Pro Pro Thr Pro Gln His Ala Pro Pro Ala Ala Thr Val His Ser Gly
```

165 170 175

Ala His Val Leu Gln Leu Gly Arg Arg Pro Ser Ala Pro Val Val Gly
180 185 190

Trp Pro Pro Val Arg Ser Phe Arg Arg Asn Leu Ala His His His His
195 200 205

Gly Ser Ser Ser Lys Gln Pro Thr Glu Pro Gln Asn Ser Glu Ala Ser
210 215 220

Arg Lys Glu Lys Pro Ala Cys Lys Lys Asn Pro Leu Val Lys Ile Asn
225 230 235 240

Met Asp Gly Ile Pro Ile Gly Arg Lys Val Asp Leu Ala Ala Tyr Asp
245 250 255

Ser Tyr Glu Arg Leu Ser Leu Gly Val Lys Glu Leu Phe His Gly Phe
260 265 270

Leu Gln Ala Gln Lys Asp Met Ser Pro Thr Ala Gly Lys Ile Phe Ser
275 280 285

Gln Leu Leu Asp Gly Ser Gly Glu Tyr Thr Leu Val Tyr Glu Asp Asn
290 295 300

Glu Gly Asp Arg Met Leu Val Gly Asp Val Pro Trp Asn Val Phe Val
305 310 315 320

Ser Thr Ala Lys Arg Leu Arg Val Leu Arg Ser Ser Glu Leu Ala Lys
325 330 335

Gly Leu Val Gly Thr Arg Pro Pro Gln Ile Ala Val Ala Pro Arg Arg
340 345 350

Gly Pro Pro Asp Cys
355

<210> 9
<211> 1468
<212> DNA
<213> Hordeum vulgare

<220>
<221> misc_feature
<222> (9)..(9)
<223> n is a, c, g, or t

<400> 9

gctgcaggna attcggcacg agggttcatc tcccgctggt gtagcatttg gaggagtccg 60

tctcggagac tttgaattat tggctcgcca ggaacaagtc gtatgggaga aaactccatg 120

gaaagagaag ccatgcctcg gctcctggat ctcatcccag acgagaaaga atggagcctg 180

agaggaggag cacctgggca cggcggatcg gaaaacacag ggttcgggag cgacgaggac 240

gagaagctgg agctcaagct tggccttcca ggccttgtaa aagaggagcc agcggccagc 300

tcaagggaca acagggtggt gcatcaggag agcccggccc tctcccttgg gtaccacccc 360

cataaacact ccacggccaa ccccacgacg accaccgggg ccaagagagg attcctggac 420

acggttgagg ccaaagcaca aggtcatgag aaggagcaga agcagcaggc gagagcagca 480

gcgtgtggga aggaactggc agcggaggaa aacacagcgg ccgcggccgt gggtgagagg 540

aagaaaggct gctgtccccc accgccgtcg catgcccctc ctgctgctgc acctgcgcgc 600

aacatcggca acagaccgca ggcgcgggga agaggggctt gcagttccag tggtcggttg 660

gcctccaatc cgatcgttca ggagaaacct tgcaagcact agtgcatcta aacaggtcca 720

tgaaccgcaa attgctgaag ccgatgccaa agcggcgctc aactgcaaga aaagccctct 780

cgtaaaaatc aacatggatg ggatccccat tggaaggaaa gttgatcttg cagcatgcga 840

cagctacgag agactttcat tggctgtcaa agatctcttc catgggtttc ttgaagtgca 900

aagggacacg cctagggctg aacgtgcaca gcaaggagca gatgaaaaaa tattttcgca 960

attgctggac gggtccggtg aatataccct agtttacgaa gacaatgaag gagacaggat 1020

gctggttggg gatgtccctt ggaatgtgtt tgtctcaacc gctaaaaggc tgagggttct 1080

gaggagctcg gagctctccc atggcctggc tggagttact ctagagagag cagcaaattg 1140

ctgaactgat ggcggctttt caaccatttg gaatgttgct cactgaagcc agatcgttat 1200

cctttttgtg gatgactagt ttagtacccc tttttttgct tgtagggacg aatgtagtga 1260

gataaattag cataccgcag atgcatggag atatcctgac tattttgtaa attaggctac 1320

cttgtattta acgctatagt tcttgactcg gtgttacatg aatttggcgt ttacaggagc 1380

tctgatggct acacctgtgc agtgcctctt ttcccggaaa aatttgtatt cagccctgat 1440

gtatcagaaa tgcaaatctg ttcaggac 1468

<210> 10
<211> 232
<212> PRT
<213> Hordeum vulgare

<400> 10

```
Met Arg Arg Ser Arg Ser Ser Arg Arg Glu Gln Gln Arg Val Gly Arg
1               5                   10                  15

Asn Trp Gln Arg Arg Lys Thr Gln Arg Pro Arg Pro Trp Val Arg Gly
            20                  25                  30

Arg Lys Ala Ala Val Pro His Arg Arg Arg Met Pro Leu Leu Leu Leu

        35                  40                  45

His Leu Arg Ala Thr Ser Ala Thr Asp Arg Arg Arg Gly Glu Glu Gly
    50                  55                  60

Leu Ala Val Pro Val Val Gly Trp Pro Pro Ile Arg Ser Phe Arg Arg
65                  70                  75                  80

Asn Leu Ala Ser Thr Ser Ala Ser Lys Gln Val His Glu Pro Gln Ile
                85                  90                  95

Ala Glu Ala Asp Ala Lys Ala Ala Leu Asn Cys Lys Lys Ser Pro Leu
            100                 105                 110

Val Lys Ile Asn Met Asp Gly Ile Pro Ile Gly Arg Lys Val Asp Leu
        115                 120                 125

Ala Ala Cys Asp Ser Tyr Glu Arg Leu Ser Leu Ala Val Lys Asp Leu
    130                 135                 140

Phe His Gly Phe Leu Glu Val Gln Arg Asp Thr Pro Arg Ala Glu Arg
145                 150                 155                 160

Ala Gln Gln Gly Ala Asp Glu Lys Ile Phe Ser Gln Leu Leu Asp Gly
                165                 170                 175

Ser Gly Glu Tyr Thr Leu Val Tyr Glu Asp Asn Glu Gly Asp Arg Met
            180                 185                 190

Leu Val Gly Asp Val Pro Trp Asn Val Phe Val Ser Thr Ala Lys Arg
        195                 200                 205

Leu Arg Val Leu Arg Ser Ser Glu Leu Ser His Gly Leu Ala Gly Val
    210                 215                 220

Thr Leu Glu Arg Ala Ala Asn Cys
225                 230
```

<210> 11

<211> 960
<212> DNA
<213> Oryza sativa

<400> 11

```
atggaagaag ggtccaacaa aagagaagga cttcctcctc aactcctaga tctcatccca      60
gatgagaagg aatggaagct gagagaagca ttaggtctag ggagatcaag aaacgcaggc     120
ttcgacggtg aagaagacaa gaagctagac ctaaagcttg gtcttcctgg ttttatagaa     180
gatgatgagg cagaaacctt aagagattac aggctacagc aagagtgccc atctctctcc     240
cttggtttct ttccaaagca ctccaagacc accagcagca ctactacgac aactggagca     300
aagaggggat tcatagacac agttgaggac aaaacagaag gttataacga ccagaagcag     360
caggcaaggg caggatgtgg gaaggaactt gcagtggagg aaatgatagc agccgtgagt     420
gagaggaaga aaggatgctg ccccccacca ccaccaccac atggtgctcc tgctacacct     480
gcgcgcaaca gaccacagac ccagggaagg agaaaccttg ctagtagtag ttcatccaaa     540
cattcccctg aaccacaaaa tgataatgcc aatgcaaagg tgacgctcac ctgcaagaaa     600
aaccctcttg taaaaatcaa catggatggg atccctattg gaaggaaaat agaccttgca     660
gcctataaca gctatgatgg gctatcgtca gctgtcaaac aactcttcca tgggtttctt     720
caagctcaaa aggaccaaac taatgcgcaa attgcacagc aaggtgcaga tgacaaaata     780
ttttatcaat tgttggatgg gtctggtgaa tatactctag tttatgaaga cagtgaagga     840
gacaggatgc tggttgggga cgtgccatgg aaagtatttg tctcaactgc taaaaggctg     900
agggttctaa ggagttcaga gctttcccat accctgattg gagctactgc tagggtctag     960
```

<210> 12
<211> 319
<212> PRT
<213> Oryza sativa

<400> 12

Met Glu Glu Gly Ser Asn Lys Arg Glu Gly Leu Pro Pro Gln Leu Leu
1 5 10 15

Asp Leu Ile Pro Asp Glu Lys Glu Trp Lys Leu Arg Glu Ala Leu Gly
20 25 30

Leu Gly Arg Ser Arg Asn Ala Gly Phe Asp Gly Glu Glu Asp Lys Lys
35 40 45

Leu Asp Leu Lys Leu Gly Leu Pro Gly Phe Ile Glu Asp Asp Glu Ala
50 55 60

Glu Thr Leu Arg Asp Tyr Arg Leu Gln Gln Glu Cys Pro Ser Leu Ser
65 70 75 80

Leu Gly Phe Phe Pro Lys His Ser Lys Thr Thr Ser Ser Thr Thr Thr
85 90 95

Thr Thr Gly Ala Lys Arg Gly Phe Ile Asp Thr Val Glu Asp Lys Thr
100 105 110

Glu Gly Tyr Asn Asp Gln Lys Gln Gln Ala Arg Ala Gly Cys Gly Lys
115 120 125

Glu Leu Ala Val Glu Glu Met Ile Ala Ala Val Ser Glu Arg Lys Lys

130 135 140

Gly Cys Cys Pro Pro Pro Pro Pro Pro His Gly Ala Pro Ala Thr Pro
145 150 155 160

Ala Arg Asn Arg Pro Gln Thr Gln Gly Arg Arg Asn Leu Ala Ser Ser
165 170 175

Ser Ser Ser Lys His Ser Pro Glu Pro Gln Asn Asp Asn Ala Asn Ala
180 185 190

Lys Val Thr Leu Thr Cys Lys Lys Asn Pro Leu Val Lys Ile Asn Met
195 200 205

Asp Gly Ile Pro Ile Gly Arg Lys Ile Asp Leu Ala Ala Tyr Asn Ser
210 215 220

Tyr Asp Gly Leu Ser Ser Ala Val Lys Gln Leu Phe His Gly Phe Leu
225 230 235 240

Gln Ala Gln Lys Asp Gln Thr Asn Ala Gln Ile Ala Gln Gln Gly Ala
245 250 255

Asp Asp Lys Ile Phe Tyr Gln Leu Leu Asp Gly Ser Gly Glu Tyr Thr
260 265 270

Leu Val Tyr Glu Asp Ser Glu Gly Asp Arg Met Leu Val Gly Asp Val
275 280 285

Pro Trp Lys Val Phe Val Ser Thr Ala Lys Arg Leu Arg Val Leu Arg
290 295 300

Ser Ser Glu Leu Ser His Thr Leu Ile Gly Ala Thr Ala Arg Val
305 310 315

<210> 13
<211> 1728
<212> DNA
<213> Triticum aestivum

<220>
<221> misc_feature
<222> (1571)..(1571)
<223> n is a, c, g, or t

<220>
<221> misc_feature
<222> (1573)..(1574)
<223> n is a, c, g, or t

<220>
<221> misc_feature
<222> (1581)..(1581)
<223> n is a, c, g, or t

<220>
<221> misc_feature
<222> (1590)..(1590)
<223> n is a, c, g, or t

<220>
<221> misc_feature
<222> (1592)..(1592)
<223> n is a, c, g, or t

<400> 13

catcctgctt ccggagctca ccatcatcgt cccttgctgg cctcaactgc tcttctcact 60
ccatctcctg catttggagt ctcggagacc ttgaattggc tcgccaagaa caagtcctat 120
ggaagaaagc tccatgaaaa gagaagccat gccccgactc ctggatctca tcccagacga 180
gaaagaatgg agtctgagag gaggagcacc agggcaggca agatcgaaaa acacaggctt 240
cgggagcgaa gaggacgaga aactggagct gaagcttggc cttccaggcc tcgtagagga 300
ggagccagcg gccagctcaa gggagaacaa cagggtgcat caggagagcc cggccctctc 360
cctcgggtac ccccctagac actccacgac catcaccacg accaccaccg gagcaaagag 420
gggattcctg gacacggttg aggccaaagc acaaggttat gagaaggagc agaagcagca 480
ggcgagggca gcagcatgtg ggaaggaact ggcagtggag gaaaatacag ctaccgtcgg 540
tgagaggaag aaagggtgct gtcccccacc accaccacca catgcccctc ctgctacacc 600
tgcgcgcaac atcggcaaca gaccgcaggc gcggggaaga ggggctgcag ctccagtggt 660
cggctggcct ccaatccggt cattcaggag aaaccttgca agcactagtg catccaaaca 720
gccccctgaa ccacaaatcg gtgaagccga tgccaaggcc gtgcttgact gcaagaaaag 780
ccctcttgta aaaatcaaca tggatgggat ccccattgga aggaaagttg atcttgcagc 840
atgtgacagc tacgggagac tttcattggc tgtcaaagat ctcttccacg ggtttcttca 900
agtgcaaagg gacccgtcga aggttgaccg tacacagcaa ggagcagatg aaaaaatatt 960
ttcgcagttg ctagacgggt ccggtgaata tactctagtt tacgaagaca gtgaaggaga 1020
caggatgctg gttggggatg tcccttggaa tgtgtttgtc tcaaccgcta aaaggctgag 1080
ggttctgaga agctcagagc tttcccatga tctgattgga gctactcctg agagagcagc 1140
aaatggctga aatggtgaca cctttcaaac catttggaat gttgctcact gaagccgtaa 1200
actaaaggaa ctcctaagaa gcaggggaga cctaaatcct gtggtcagtt tggagatcgt 1260
tatccttttt gaggattagt agttcggtaa cctttttttt tcttgtagtg agatgaatta 1320
gcataccgca gatgcatgga gatatcctgc ctattttgta aattaggcta ccttgtattt 1380
aacgctatag ttctcgattc agttacatag atttggcgcc tattttctct gacaaatttg 1440
taacctgaca tgcaaatctg ttcatgacaa aagaggacca gcacctggcc tgtcctgata 1500
gcatcacacc accgaacgat atatatggag tagcaacttc atgtggaata gctataaagt 1560
tcagggtttt ncnnaaaaca nacccccccn cnaaaaaaag ggcgggcgct taaaatatcc 1620
cagaggggcc agtttcccgt cctcgttttt gataaaaggg gcccatatgg ggtctataaa 1680
aagtaggccg ggccgggttt aaaacgttgt aatggaaaac tggtacta 1728

<210> 14
<211> 343
<212> PRT

<213> Triticum aestivum

<400> 14

```
Met Glu Glu Ser Ser Met Lys Arg Glu Ala Met Pro Arg Leu Leu Asp
1               5                   10                  15

Leu Ile Pro Asp Glu Lys Glu Trp Ser Leu Arg Gly Gly Ala Pro Gly
            20                  25                  30

Gln Ala Arg Ser Lys Asn Thr Gly Phe Gly Ser Glu Glu Asp Glu Lys
        35                  40                  45

Leu Glu Leu Lys Leu Gly Leu Pro Gly Leu Val Glu Glu Glu Pro Ala
    50                  55                  60

Ala Ser Ser Arg Glu Asn Asn Arg Val His Gln Glu Ser Pro Ala Leu
65                  70                  75                  80

Ser Leu Gly Tyr Pro Pro Arg His Ser Thr Thr Ile Thr Thr Thr Thr
                85                  90                  95

Thr Gly Ala Lys Arg Gly Phe Leu Asp Thr Val Glu Ala Lys Ala Gln
            100                 105                 110

Gly Tyr Glu Lys Glu Gln Lys Gln Gln Ala Arg Ala Ala Ala Cys Gly
        115                 120                 125

Lys Glu Leu Ala Val Glu Glu Asn Thr Ala Thr Val Gly Glu Arg Lys
    130                 135                 140

Lys Gly Cys Cys Pro Pro Pro Pro Pro Pro His Ala Pro Pro Ala Thr
145                 150                 155                 160

Pro Ala Arg Asn Ile Gly Asn Arg Pro Gln Ala Arg Gly Arg Gly Ala
                165                 170                 175

Ala Ala Pro Val Val Gly Trp Pro Pro Ile Arg Ser Phe Arg Arg Asn
            180                 185                 190

Leu Ala Ser Thr Ser Ala Ser Lys Gln Pro Pro Glu Pro Gln Ile Gly
        195                 200                 205
```

Glu Ala Asp Ala Lys Ala Val Leu Asp Cys Lys Lys Ser Pro Leu Val
    210                 215                 220

Lys Ile Asn Met Asp Gly Ile Pro Ile Gly Arg Lys Val Asp Leu Ala
225                 230                 235                 240

Ala Cys Asp Ser Tyr Gly Arg Leu Ser Leu Ala Val Lys Asp Leu Phe
                245                 250                 255

His Gly Phe Leu Gln Val Gln Arg Asp Pro Ser Lys Val Asp Arg Thr
            260                 265                 270

Gln Gln Gly Ala Asp Glu Lys Ile Phe Ser Gln Leu Leu Asp Gly Ser
        275                 280                 285

Gly Glu Tyr Thr Leu Val Tyr Glu Asp Ser Glu Gly Asp Arg Met Leu
    290                 295                 300

Val Gly Asp Val Pro Trp Asn Val Phe Val Ser Thr Ala Lys Arg Leu
305                 310                 315                 320

Arg Val Leu Arg Ser Ser Glu Leu Ser His Asp Leu Ile Gly Ala Thr
                325                 330                 335

Pro Glu Arg Ala Ala Asn Gly
            340

<210> 15
<211> 1689
<212> DNA
<213> Triticum aestivum

<400> 15

```
ctcgcgctca aagcctggtc ggtaacagag ccaaggtgcc atttctcgac ccccctctcc     60
acatacctct cctagctagc tacccatccg cttccacttg gccatttccc atcccacatc    120
ctgcttgctt ccggagctca tcagcatcgc ttggcctaag gctcgcttca actgcctgct    180
cttctcagct catcccctgc atttggagtc tcggagactt tgaattggct cgccaagaac    240
gagtcctatg gaagaaagct ccatgaaaag agaagccatg cctcgactcc tggatctcat    300
cccggacgag aaagaatgga gcctgagagg aggagcgcca gggcagggca ggtcaaaaaa    360
cacaggcttc gggagcgacg aggacgagaa actggagctg aagcttggcc ttccaggcct    420
cgtacaagag gagccagcgg ccagctcaag agagaagagg gtgcatcagg agagcccggc    480
cctctacctt gggtaccccc ctagacactc cacggccacc acaacgacga ccaccaccgg    540
agcaaagagg gggttcctgg acacggttga ggccaaagca caaggttatg agaaggagca    600
ggcgagagca gcaacatgtg ggaaggaact ggcgatggag gaaaatacag aggccgtcgg    660

tgagaggaag aaagggtgct gtcccccacc accaccagca catgcccctc ctgctacacc    720
tgcgcgcaac atcggcaaca gaccgcaggc gcggggaaga ggggctgcag ctccagtggt    780
cggctggcct ccaatccggt cattcaggag aaaccttgca agcactagtg catccaaaca    840
gccccctgaa ccgcaaatcg gtgaagccaa tgccaaggcc gttcttgact gcaagaaaag    900
ccctcttgta aaaatcaaca tggatgggat ccccattgga aggaaagttg atcttgcagc    960
atgtgacagc tacgagagac tttcattggc tgtcaaagat ctcttccacg ggtttcttca   1020
agtgcaaagg gacccgtcta aggttgaacg tacacagcaa ggagcagatg aaaatatatt   1080
ttcgcggttg ctagacgggt ccggtgaata tactctagtt tacgaagaca gtgaaggaga   1140
caggatgctg gttggggatg tcccttggaa tgtgtttgtc tcgaccgcta aaaggctgag   1200
ggttctgaga agctcagagc tttcccatgg tctgattgga gctactcctg agagaacagc   1260
acatggctga aatggtgaca ccttttcaac catttgaaat gttgctcact gaagccggaa   1320
actaaatgaa ctcctaacaa gcaggggaat gtctcctttg ttactggtcg ggaagaccta   1380
aatcctgtgg tcagttcgga gatcgttgtc ctttttgagg attagtagtt cagtaccctt   1440
tttttttgct tgtagtgaga tgaattagca taccgcagat gcatggagat atcctgccta   1500
ttttgtaaat taggctacct tgtatttaac gctatagttc ttgattcagt tacacagatt   1560
cggtgcctac tttctctgac aaaatttgta acctgacatg caaatctgtt caggacaaaa   1620
gaggaccagc acctggcctg tcctgatagc atcacaccac cgaacgatat ataggattag   1680
caacttcat                                                           1689
```

<210> 16
<211> 340

<212> PRT
<213> Triticum aestivum

<400> 16

```
Met Glu Glu Ser Ser Met Lys Arg Glu Ala Met Pro Arg Leu Leu Asp
1               5                   10                  15

Leu Ile Pro Asp Glu Lys Glu Trp Ser Leu Arg Gly Gly Ala Pro Gly
            20                  25                  30

Gln Gly Arg Ser Lys Asn Thr Gly Phe Gly Ser Asp Glu Asp Glu Lys
        35                  40                  45

Leu Glu Leu Lys Leu Gly Leu Pro Gly Leu Val Gln Glu Glu Pro Ala
    50                  55                  60

Ala Ser Ser Arg Glu Lys Arg Val His Gln Glu Ser Pro Ala Leu Tyr
65                  70                  75                  80
```

```
Leu Gly Tyr Pro Pro Arg His Ser Thr Ala Thr Thr Thr Thr Thr Thr
                85                  90                  95

Thr Gly Ala Lys Arg Gly Phe Leu Asp Thr Val Glu Ala Lys Ala Gln
            100                 105                 110

Gly Tyr Glu Lys Glu Gln Ala Arg Ala Ala Thr Cys Gly Lys Glu Leu
        115                 120                 125

Ala Met Glu Glu Asn Thr Glu Ala Val Gly Glu Arg Lys Lys Gly Cys
    130                 135                 140

Cys Pro Pro Pro Pro Pro Ala His Ala Pro Pro Ala Thr Pro Ala Arg
145                 150                 155                 160

Asn Ile Gly Asn Arg Pro Gln Ala Arg Gly Arg Gly Ala Ala Ala Pro
                165                 170                 175

Val Val Gly Trp Pro Pro Ile Arg Ser Phe Arg Arg Asn Leu Ala Ser
            180                 185                 190

Thr Ser Ala Ser Lys Gln Pro Pro Glu Pro Gln Ile Gly Glu Ala Asn
        195                 200                 205

Ala Lys Ala Val Leu Asp Cys Lys Lys Ser Pro Leu Val Lys Ile Asn
    210                 215                 220

Met Asp Gly Ile Pro Ile Gly Arg Lys Val Asp Leu Ala Ala Cys Asp
225                 230                 235                 240

Ser Tyr Glu Arg Leu Ser Leu Ala Val Lys Asp Leu Phe His Gly Phe
                245                 250                 255

Leu Gln Val Gln Arg Asp Pro Ser Lys Val Glu Arg Thr Gln Gln Gly
            260                 265                 270

Ala Asp Glu Asn Ile Phe Ser Arg Leu Leu Asp Gly Ser Gly Glu Tyr
        275                 280                 285

Thr Leu Val Tyr Glu Asp Ser Glu Gly Asp Arg Met Leu Val Gly Asp
    290                 295                 300

Val Pro Trp Asn Val Phe Val Ser Thr Ala Lys Arg Leu Arg Val Leu
305                 310                 315                 320

Arg Ser Ser Glu Leu Ser His Gly Leu Ile Gly Ala Thr Pro Glu Arg
                325                 330                 335

Thr Ala His Gly
```

340

<210> 17
<211> 1273
<212> DNA
<213> Triticum aestivum

<400> 17

```
aggccagctc gagcaaccac aacgagccgc cgacgccgac gccgtgcgtg gtctcgccat     60
tacttcctcg agctctccat cccggccgcg gccaggactc ctccggttgc agccctgctg    120
cagctgtccg acccccgtct ccggtgcggc acgtctagat ccagtccaat ggaacaaggc    180
acccctcccc ggctgctgaa cctcatcccg gatgacaagg ggtggcaggc gagggaagct    240
caagcaggcc accgaggaac aaggccacca ttcgacgccg agcacgagca cgagcgcgag    300
gaggacaagg agctggacct caagctcggc ctccccggag tccagcacca aggagagagc    360
tgctcggcgg tctccctggc gcgcttcgcc acgcattcat gcctcaccag caccaccacc    420
ggcgccaaga gaggcttctt cgacgccgtt ggggccaaac cacaaggtta tgatcggagg    480
cagcaagagg acagggaagg acgtgcacct gtgcacagca gtagagaccc cccggggaga    540
gctgctgttg ttccggtagt cggctggcct ccagtccgat ccttcaggag aaacctcaca    600
aatggaactt catctaagca acaatcccct gagcgacaaa aacgtcgagg ccaccgacag    660
agcgaagccg gtctgcaaga agaaaccgct catcaagatc aacatggatg gaatacctat    720
tggaagaaaa gtagacctcg catcatgtga caactaccat aagctgtcga gtgccgtcga    780
agagttgttc cgaggctttc ttgaagccca gcaggattta tcttgtgatg aaagcggagt    840
gcgaggagca gaagagaaat tattttcagg gttgctggat ggaaccggtg aatacgctct    900
agtgtacgaa gatgacgaag gggacaggat gctggtcggg gacataccat ggagtgtgtt    960
tgtttcgact gcgaagagac tgagggtcat gaggaggtca gagcttcccc aacgcatgac   1020
tggagctaac tcaacgaaag tagcagattg ctgaaaatga gggtgcatgc atttccatgg   1080
cacagaccgc agttcatcac atttttggag atcgcccgtc tttagctcac agtgatgaat   1140
ctaaaaagga ggaaaaaaac atctcgtagt tgcagctaca gtgggcacac tgcatatctt   1200
gtaaattacc ctactctttg tttgaatgta gtgttggtca ttgcaatagt acatgttgca   1260
gtaattgcct ttg                                                      1273
```

<210> 18
<211> 150
<212> PRT
<213> Triticum aestivum

<400> 18

```
Met Glu Leu His Leu Ser Asn Asn Pro Leu Ser Asp Lys Asn Val Glu
1               5                   10                  15

Ala Thr Asp Arg Ala Lys Pro Val Cys Lys Lys Lys Pro Leu Ile Lys
            20                  25                  30

Ile Asn Met Asp Gly Ile Pro Ile Gly Arg Lys Val Asp Leu Ala Ser
        35                  40                  45

Cys Asp Asn Tyr His Lys Leu Ser Ser Ala Val Glu Glu Leu Phe Arg
    50                  55                  60

Gly Phe Leu Glu Ala Gln Gln Asp Leu Ser Cys Asp Glu Ser Gly Val
65                  70                  75                  80

Arg Gly Ala Glu Glu Lys Leu Phe Ser Gly Leu Leu Asp Gly Thr Gly
                85                  90                  95

Glu Tyr Ala Leu Val Tyr Glu Asp Asp Glu Gly Asp Arg Met Leu Val
            100                 105                 110

Gly Asp Ile Pro Trp Ser Val Phe Val Ser Thr Ala Lys Arg Leu Arg
        115                 120                 125

Val Met Arg Arg Ser Glu Leu Pro Gln Arg Met Thr Gly Ala Asn Ser
    130                 135                 140

Thr Lys Val Ala Asp Cys
145                 150
```

<210> 19
<211> 1340
<212> DNA
<213> Zea mays

<400> 19

agtgccttac aacactccat ccatctccat gggagactcc ggaggaagaa gaggaccacc 60
tcttcctcgg ctgctagatc tcatcccgga cgggaaagaa cggaagggga gggaagcgca 120
agacgcggga aggtcgaaga acagcggcgg cttcgggggc gaggaggacg cgaagctgga 180
gctcaagctc gggcctccgg gtctcactct cgtagaagaa ggaacgacag ctaccgtgcc 240
aagagatgga ggcgtactac agcgagagac gacgaccacc ccgaccctct cgctcgggtg 300
cttcccacgt aacccctcca agcctgcggt agacgcagct agcagctggg acgaagaggg 360
ggttcttacg ccaccgccgt cgaggccaag acagaaggtc cgcgacgagc ggatggagca 420
gcaggccgga gctggatgtg ggaacgagct agcactggac gagaagacgg cagccgcggc 480
cgcgagtgag agacagaaag ggtcctgctg cccgccgacg ccacagcacg cccctcctgc 540
tgcgaccgtg cacagcggag cacacgtcct gcagctggga agaaggcctt cggctccggt 600
tgtcggttgg cccccggtca gatccttcag gagaaacctt gctcatcatc atcatggaag 660
ctcctccaaa caacccaccg agccacagaa cagcgaagct agcaggaagg agaagcctgc 720
ctgcaagaag aaccctctcg taaaaatcaa catggacggg atccccatcg gaaggaaagt 780
agaccttgca gcctacgaca gctacgagag gctatcactg ggcgtcaaag agcttttcca 840
cggttttctt caagctcaaa aggatatgtc tccaactgct ggaaagatat tctctcaatt 900
gttggatgga tctggcgaat ataccctggt ctacgaagac aacgaaggag acaggatgct 960
ggtaggggac gtgccgtgga atgtgtttgt gtcaactgct aaaaggctga gggttttgag 1020
gagctcggag cttgccaaag gtttggtagg cacaaggcca cctcagattg cggtagctcc 1080
gagaagagga ccaccagatt gctgaaagcc tgaaacagcg acaaaattct gatcatttgg 1140
aatgtgagca agcaaactga acccggacct tttgtgatgt gacggtactt ttttcttttt 1200
gcctgtagaa actaatctag tgagatgaac tgccatttca gatgcatgcc gaggtcctgg 1260
atattttgta aattaggcta ccaataccat gcgactcgtt gctgtagagt aataatgtcc 1320
tgatgtaatt caacatgggc 1340

<210> 20
<211> 358
<212> PRT
<213> Zea mays

<400> 20

```
Met Gly Asp Ser Gly Gly Arg Arg Gly Pro Pro Leu Pro Arg Leu Leu
1               5                   10                  15

Asp Leu Ile Pro Asp Gly Lys Glu Arg Lys Gly Arg Glu Ala Gln Asp
            20                  25                  30

Ala Gly Arg Ser Lys Asn Ser Gly Gly Phe Gly Gly Glu Glu Asp Ala
        35                  40                  45

Lys Leu Glu Leu Lys Leu Gly Pro Pro Gly Leu Thr Leu Val Glu Glu
    50                  55                  60

Gly Thr Thr Ala Thr Val Pro Arg Asp Gly Gly Val Leu Gln Arg Glu
65                  70                  75                  80

Thr Thr Thr Thr Pro Thr Leu Ser Leu Gly Cys Phe Pro Arg Asn Pro
                85                  90                  95

Ser Lys Pro Ala Val Asp Ala Ala Ser Ser Trp Asp Glu Glu Gly Val
            100                 105                 110

Leu Thr Pro Pro Pro Ser Arg Pro Arg Gln Lys Val Arg Asp Glu Arg
        115                 120                 125

Met Glu Gln Gln Ala Gly Ala Gly Cys Gly Asn Glu Leu Ala Leu Asp
    130                 135                 140
```

```
Glu Lys Thr Ala Ala Ala Ala Ala Ser Glu Arg Gln Lys Gly Ser Cys
145                 150                 155                 160

Cys Pro Pro Thr Pro Gln His Ala Pro Pro Ala Ala Thr Val His Ser
                165                 170                 175

Gly Ala His Val Leu Gln Leu Gly Arg Arg Pro Ser Ala Pro Val Val
            180                 185                 190

Gly Trp Pro Pro Val Arg Ser Phe Arg Arg Asn Leu Ala His His His
        195                 200                 205

His Gly Ser Ser Ser Lys Gln Pro Thr Glu Pro Gln Asn Ser Glu Ala
    210                 215                 220

Ser Arg Lys Glu Lys Pro Ala Cys Lys Lys Asn Pro Leu Val Lys Ile
225                 230                 235                 240

Asn Met Asp Gly Ile Pro Ile Gly Arg Lys Val Asp Leu Ala Ala Tyr
                245                 250                 255

Asp Ser Tyr Glu Arg Leu Ser Leu Gly Val Lys Glu Leu Phe His Gly
            260                 265                 270

Phe Leu Gln Ala Gln Lys Asp Met Ser Pro Thr Ala Gly Lys Ile Phe
        275                 280                 285

Ser Gln Leu Leu Asp Gly Ser Gly Glu Tyr Thr Leu Val Tyr Glu Asp
    290                 295                 300

Asn Glu Gly Asp Arg Met Leu Val Gly Asp Val Pro Trp Asn Val Phe
305                 310                 315                 320

Val Ser Thr Ala Lys Arg Leu Arg Val Leu Arg Ser Ser Glu Leu Ala
                325                 330                 335

Lys Gly Leu Val Gly Thr Arg Pro Pro Gln Ile Ala Val Ala Pro Arg
            340                 345                 350

Arg Gly Pro Pro Asp Cys
        355
```

<210> 21
<211> 159
<212> PRT
<213> Artificial sequence

<220>

<223> AUX/IAA domain: consensus sequence

<220>
<221> misc_feature
<222> (22)..(25)
<223> Xaa can be any naturally occurring amino acid

<220>
<221> misc_feature
<222> (35)..(35)
<223> Xaa can be any naturally occurring amino acid

<220>
<221> misc_feature
<222> (38)..(38)
<223> Xaa can be any naturally occurring amino acid

<220>
<221> misc_feature
<222> (44)..(44)
<223> Xaa can be any naturally occurring amino acid

<220>
<221> misc_feature
<222> (48)..(48)
<223> Xaa can be any naturally occurring amino acid

<220>
<221> misc_feature
<222> (93)..(93)
<223> Xaa can be any naturally occurring amino acid

<220>
<221> misc_feature
<222> (95)..(95)
<223> Xaa can be any naturally occurring amino acid

<220>
<221> misc_feature
<222> (98)..(98)
<223> Xaa can be any naturally occurring amino acid

<400> 21

```
Ala Pro Val Val Gly Trp Pro Pro Ile Arg Ser Phe Arg Arg Asn Leu
1               5                   10                  15

Ala Ser Thr Ser Ser Xaa Xaa Xaa Xaa Ser Lys Gln Pro Pro Glu Pro
            20                  25                  30

Gln Asn Xaa Glu Ala Xaa Ala Lys Ala Lys Leu Xaa Cys Lys Lys Xaa
        35                  40                  45

Pro Leu Val Lys Ile Asn Met Asp Gly Ile Pro Ile Gly Arg Lys Val
    50                  55                  60

Asp Leu Ala Ala Tyr Asp Ser Tyr Glu Arg Leu Ser Ser Ala Val Lys
65                  70                  75                  80

Asp Leu Phe His Gly Phe Leu Gln Ala Gln Lys Asp Xaa Ser Xaa Ala

                85                  90                  95

Glu Xaa Ala Gln Gln Gly Ala Asp Glu Lys Ile Phe Ser Gln Leu Leu
            100                 105                 110

Asp Gly Ser Gly Glu Tyr Thr Leu Val Tyr Glu Asp Ser Glu Gly Asp
        115                 120                 125

Arg Met Leu Val Gly Asp Val Pro Trp Asn Val Phe Val Ser Thr Ala
    130                 135                 140

Lys Arg Leu Arg Val Leu Arg Ser Ser Glu Leu Ser His Gly Leu
145                 150                 155
```

<210> 22
<211> 6
<212> PRT
<213> Artificial sequence

<220>
<223> motif 1

<220>
<221> VARIANT
<222> (1)..(1)
<223> / replace = "Asn"

<220>
<221> VARIANT
<222> (2).. (2)
<223> / replace = "Met"

<220>

<221> VARIANT
<222> (4)..(4)
<223> / replace = "Trp"

<220>
<221> VARIANT
<222> (5)..(5)
<223> / replace = "Gly"

<400> 22

```
Lys Ile Phe Ser Gln Leu
1               5
```

<210> 23
<211> 11
<212> PRT
<213> Artificial sequence

<220>
<223> motif 2

<220>
<221> VARIANT
<222> (1)..(1)
<223> / replace = "Gly"

<220>
<221> VARIANT
<222> (6)..(6)
<223> / replace = "Thr"

<220>
<221> VARIANT
<222> (8)..(8)
<223> / replace = "Val"

<400> 23

```
Gln Leu Leu Asp Gly Ser Gly Glu Tyr Thr Leu
1               5                   10
```

<210> 24
<211> 12
<212> PRT
<213> Artificial sequence

<220>
<223> motif 3

<400> 24

```
Leu Leu Asp Gly Ser Gly Glu Tyr Thr Leu Val Tyr
1               5                   10
```

<210> 25
<211> 11
<212> PRT

<213> Artificial sequence

<220>
<223> motif 4

<400> 25

```
Lys Lys Leu Glu Leu Arg Leu Gly Pro Pro Gly
1               5                   10
```

<210> 26
<211> 2194
<212> DNA
<213> Oryza sativa

<400> 26

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
```

atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc tttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctccttctc ccatctataa attcctcccc ccttttcccc tctctatata 1020
ggaggcatcc aagccaagaa gagggagagc accaaggaca cgcgactagc agaagccgag 1080
cgaccgcctt ctcgatccat atcttccggt cgagttcttg gtcgatctct tccctcctcc 1140
acctcctcct cacagggtat gtgcctccct tcggttgttc ttggatttat tgttctaggt 1200
tgtgtagtac gggcgttgat gttaggaaag gggatctgta tctgtgatga ttcctgttct 1260
tggatttggg atagaggggt tcttgatgtt gcatgttatc ggttcggttt gattagtagt 1320
atggttttca atcgtctgga gagctctatg gaaatgaaat ggtttaggga tcggaatctt 1380
gcgattttgt gagtaccttt tgtttgaggt aaaatcagag caccggtgat tttgcttggt 1440
gtaataaagt acggttgttt ggtcctcgat tctggtagtg atgcttctcg atttgacgaa 1500
gctatccttt gtttattccc tattgaacaa aaataatcca actttgaaga cggtcccgtt 1560
gatgagattg aatgattgat tcttaagcct gtccaaaatt tcgcagctgg cttgtttaga 1620
tacagtagtc cccatcacga aattcatgga aacagttata atcctcagga acaggggatt 1680
ccctgttctt ccgatttgct ttagtcccag aattttttt cccaaatatc ttaaaaagtc 1740
actttctggt tcagttcaat gaattgattg ctacaaataa tgcttttata gcgttatcct 1800
agctgtagtt cagttaatag gtaatacccc tatagtttag tcaggagaag aacttatccg 1860
atttctgatc tccattttta attatatgaa atgaactgta gcataagcag tattcatttg 1920
gattattttt tttattagct ctcacccctt cattattctg agctgaaagt ctggcatgaa 1980
ctgtcctcaa ttttgttttc aaattcacat cgattatcta tgcattatcc tcttgtatct 2040
acctgtagaa gtttcttttt ggttattcct tgactgcttg attacagaaa gaaatttatg 2100
aagctgtaat cgggatagtt atactgcttg ttcttatgat tcatttcctt tgtgcagttc 2160
ttggtgtagc ttgccacttt caccagcaaa gttc 2194

## Description

The present invention relates generally to the field of molecular biology and concerns a method for increasing seed yield in plants. More specifically, the present invention concerns a method for increasing seed yield in plants by introduction and overexpression in a plant of a nucleic acid encoding an IAA2-like (auxin/indoleacetic acid 2 like) polypeptide. The present invention also concerns plants having increased expression of a nucleic acid encoding IAA2-like polypeptide, which plants have increased seed yield relative to control plants. The invention also provides constructs comprising IAA2-like-encoding nucleic acids, useful in performing the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

A further important trait is that of improved abiotic stress tolerance. Abiotic stress is a primary cause of crop loss worldwide, reducing average yields for most major crop plants by more than 50% (Wang et al., Planta 218, 1-14, 2003). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defence mechanisms.

It has now been found that seed yield may be improved in plants by introduction and overexpression in a plant of a nucleic acid encoding an IAA2-like polypeptide.

**Background**

The AUX/IAA (auxin/indoleacetic acid) genes encode a family of proteins whose expression is tightly regulated by auxin. The plant hormone auxin is involved in various processes like cell division, cell expansion and differentiation, patterning of embryos, vasculature or other tissues, regulation of growth of primary and lateral root or shoot meristems. AUX/IAA proteins furthermore are usually expressed in a tissue-specific manner.

AUX/IAA proteins typically have four conserved amino acid sequence motifs (domains I, II, III and IV) and have nuclear localisation signal sequences. Domains I and II are postulated to destabilize the protein and may be involved in protein turnover. Domains III and IV are postulated to be involved in protein-protein interactions: AUX/IAA proteins can form homodimers and are known to associate with ARF proteins. The AUX/IAA-ARF complexes are likely to be involved in auxin mediated gene expression. The Aux/IAA proteins are negative regulators of the auxin response factors (ARFs) that regulate expression of auxin-responsive genes. Aux/IAA proteins bind to the DNA-bound ARF partner proteins and repress ARF activity. In the auxin activated status, Aux/IAA proteins are ubiquitinated via interactions with the auxin-modified SCFTIR1complex and subsequently degraded by 26S proteasome action. An overview of roles and activities of AUX/IAA proteins is given by Reed (Trends in Plant Science 6, 420-425, 2001). The structure and expression analysis of early auxin-responsive Aux/IAA gene family in rice *(Oryza sativa*) has recently been reported by Jain et al. 2006 Funct Integr Genomics. 2006 Jan;6(1):47-59.

Published US patent application, US 2004/123343, in the name of La Rosa, describes almost 205,000 polypeptide and polynucleotide sequences, including a sequence comprising an AUX/IAA domain.

Published PCT patent application, WO 03/008540, in the name of Syngenta, discloses clusters of genes, identified using microarrays, the expression of which is altered by stress conditions. Several thousand such nucleotide and protein sequences are disclosed in this prior art document, including a sequence comprising an AUX/IAA domain.

Published PCT patent application, WO 2007/117693, in the name of Ceres, concerns methods for the identification of regulatory regions capable of interacting with regulatory proteins involved in alkaloid biosynthesis, which enables the production of plants having modified alkaloid content. One such sequence disclosed in this prior art document is a sequence comprising an AUX/IAA domain.

Published PCT patent application, WO 2009/016232, in the name of BASF Plant Science GmbH and The Crop Functional Genomics Center, concerns a method for increasing seed yield in plants by introducing and overexpressing a nucleic acid encoding a PEPC or PQQC protein.

The promoter for the rice gene *GOS2* is disclosed in De Pater et al., 1992, (The Plant Journal, 2(6), 837-844).

## Summary

Surprisingly, it has now been found that introduction and overexpression in a plant of a nucleic acid encoding an IAA2-like polypeptide gives plants having increased seed yield relative to control plants.

According to one embodiment, there is provided a method for increasing plant seed yield relative to control plants, comprising introduction and overexpression of a nucleic acid encoding an IAA2-like polypeptide in a plant.

## Definitions

<u>Polypeptide(s)/Protein(s)</u>

The terms "polypeptide" and "protein" are used interchangeably herein and refer to amino acids in a polymeric form of any length, linked together by peptide bonds.

<u>Polynucleotide(s)/Nucleic acid(s)/Nucleic acid sequence(s)/nucleotide sequence(s)</u>

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

<u>Homologue(s)</u>

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheer structures). Amino acid substitutions are typically of single residues, but may be clustered depending upon functional constraints placed upon the polypeptide and may range from 1 to 10 amino acids; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain, Motif/Consensus sequence/Signature

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo, Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

Reciprocal BLAST

Typically, this involves a first BLAST involving BLASTing a query sequence (for example using any of the sequences listed in Table A of the Examples section) against any sequence database, such as the publicly available NCBI database. BLAST or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived. The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point ($T_m$) for the specific sequence at a defined ionic strength and pH. Medium stringency conditions are when the temperature is 20°C below $T_m$, and high stringency conditions are when the temperature is 10°C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16°C up to 32°C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6x\log_{10}[Na^+]^a + 0.41x\%[G/C^b] - 500x[L^c]^{-1} - 0.61x\% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

a or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

Construct

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |

(continued)

| Gene Source | Reference |
|---|---|
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-regulated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b:** Examples of root-specific promoters

| Gene Source | Reference |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161 (2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |

(continued)

| Gene Source | Reference |
|---|---|
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2c to Table 2f below. Further examples of seed-specific promoters are given in Qing Qu and Takaiwa (Plant Biotechnol. J. 2, 113-125, 2004).

**Table 2c:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat $\alpha$, $\beta$, $\gamma$-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice $\alpha$-globulin Glb-1 | Wu et al, Plant Cell Physiology 39(8) 885-889, 1998 |

(continued)

| Gene source | Reference |
|---|---|
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d:** examples of endosperm-specific promoters

| Gene source | Reference |
|---|---|
| glutelin (rice) | Takaiwa et al. (1986) Mol Gen Genet 208:15-22;<br>Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90,<br>Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley Itr1 promoter | Diaz et al. (1995) Mol Gen Genet 248(5):592-8 |
| barley B1, C, D, hordein | Cho et al. (1999) Theor Appl Genet 98:1253-62;<br>Muller et al. (1993) Plant J 4:343-55;<br>Sorenson et al. (1996) Mol Gen Genet 250:750-60 |
| barley DOF | Mena et al, (1998) Plant J 116(1): 53-62 |

(continued)

| Gene source | Reference |
|---|---|
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e**: Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f**: Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g below.

**Table 2g:** Examples of green tissue-specific promoters

| Gene | Expression | Reference |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |

(continued)

| Gene | Expression | Reference |
|---|---|---|
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h:** Examples of meristem-specific promoters

| Gene source | Expression pattern | Reference |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

<u>Terminator</u>

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the IoxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA. transcripts to be translated into polypeptides. For further general details see for example, Grierson et al. (1998) WO 98/53083; Waterhouse et al. (1999) WO 99/53050).

Performance of the methods does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

The antisense nucleic acid sequence can be an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

## Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R.

Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

T-DNA activation tagging

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8), and approaches exist that are generally applicable regardless of the target organism (Miller et al, Nature Biotechnol. 25, 778-785, 2007).

Yield related Traits

Yield related traits comprise one or more of yield, biomass, seed yield, early vigour, greenness index, increased growth rate, improved agronomic traits (such as improved Water Use Efficiency (WUE), Nitrogen Use Efficiency (NUE), etc.).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increased growth rate

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Stress resistance

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35%, 30% or 25%, more preferably less than 20% or 15% in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Nutrient deficiency may result from a lack of nutrients such as nitrogen, phosphates and other phosphorous-containing compounds, potassium, calcium, magnesium, manganese, iron and boron, amongst others.

The term salt stress is not restricted to common salt (NaCl), but may be any one or more of: NaCl, KCl, LiCl, $MgCl_2$, $CaCl_2$, amongst others.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Marker assisted breeding

Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Use as probes in (gene mapping)

Use of nucleic acids encoding the protein of interest for genetically and physically mapping the genes requires only a nucleic acid sequence of at least 15 nucleotides in length. These nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids encoding the protein of interest. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding the protein of interest in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp.,

*Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

**Detailed description of the invention**

Surprisingly, it has now been found that introduction and overexpression in a plant of a nucleic acid encoding an IAA2-like polypeptide gives plants having increased seed yield relative to control plants. According to a first embodiment, the present invention provides a method for increasing seed yield in plants relative to control plants, comprising introduction and overexpression in a plant of a nucleic acid encoding an IAA2-like polypeptide.

Described herein are methods for modulating (preferably, increasing) expression of a nucleic acid encoding an IAA2-like polypeptide by introducing and overexpressing in a plant a nucleic acid encoding an IAA2-like polypeptide.

Any reference hereinafter to a "protein" or "polypeptide" "useful in the methods of the invention" is taken to mean an IAA2-like polypeptide represented by SEQ ID NO: 2 or having at least 80% overall sequence identity to SEQ ID NO: 2. Any reference hereinafter to a "nucleic acid useful in the methods of the invention" is taken to mean a nucleic acid capable of encoding such an IAA2-like polypeptide. The nucleic acid to be introduced into a plant (and therefore useful in performing the methods of the invention) is any nucleic acid encoding an IAA2-like polypeptide represented by SEQ ID NO: 2 or having at least 80% overall sequence identity to SEQ ID NO: 2, hereinafter also named *"IAA2-like* nucleic acid" or *"IAA2-like* gene".

Described herein are IAA2-like polypeptides comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

Also described are IAA2-like polypeptides further comprising one of more of the following motifs:

Motif 2, SEQ ID NO: 23: (Q/G)LLDG(S/T)G(E/V)YTL
Motif 3, SEQ ID NO: 24: LLDGSGEYTLVY
Motif 4, SEQ ID NO: 25: KKLELRLGPPG,

wherein amino acids indicated between brackets at a position represent alternative amino acids at that position.

The AUX/IAA domain spans amino acids 49 to 335 in SEQ ID NO: 2. A consensus sequence comprising the most highly conserved amino acids in IAA2-like polypeptide may be represented by SEQ ID NO: 21.

In addition, the IAA2-like polypeptide may have in increasing order of preference at least 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid sequence of the AUX/IAA domain of any of the polypeptides of Table A1, preferably the amino acid sequence of the AUX/IAA domain of SEQ ID NO: 2, even more preferably to the sequence of the consensus AUX/IAA domain in IAA2-like polypeptides as represented by SEQ ID NO: 21.

Alternatively, the homologue of an IAA2-like protein may have in increasing order of preference at least 80%, 81%, 82%, 83%, 84%, 85%, 86%, 87%, 88%, 89%, 90%, 91%, 92%, 93%, 94%, 95%, 96%, 97%, 98%, or 99% overall sequence identity to the amino acid represented by any of the polypeptide sequences of Table A1, preferably by SEQ ID NO: 2, provided that the homologous protein comprises an AUX/IAA domain and one or more of the conserved motifs as outlined above. The overall sequence identity is determined using a global alignment algorithm, such as the Needleman Wunsch algorithm in the program GAP (GCG Wisconsin Package, Accelrys), preferably with default parameters and preferably with sequences of mature proteins (i.e. without taking into account secretion signals or transit peptides). Compared to overall sequence identity, the sequence identity will generally be higher when only conserved domains or motifs are considered.

The terms "domain", "signature" and "motif" are defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics; Gasteiger et al., Nucleic Acids Res. 31:3784-3788 (2003)). Domains or motifs may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters. For local alignments, the Smith-Waterman algorithm is particularly useful (Smith TF, Waterman MS (1981) J. Mol. Biol 147(1);195-7).

The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labour-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others.

Furthermore, IAA2-like polypeptides (at least in their native form) typically have protein binding activity. Tools and techniques for measuring protein binding activity are well known in the art and include for example, inmunoprecipitation of protein complexes or yeast two hybrid. Tools and techniques for measuring the association of IAA and ARF polypeptides are well known in the art., and include for example yeast two hybrid analysis (see for example Fukaki et al. (Plant J. 44, 382-395, 2005).

In addition, IAA2-like polypeptides, when expressed in rice according to the methods of the present invention, as outlined in the Examples section, give plants having increased seed yield

In addition, IAA2-like polypeptides, when expressed in rice according to the methods of the present invention, as outlined in the Examples section, give plants having increased seed fill rate, thousand kernel weight (TKW) and increased harvest index.

Additionally, IAA2-like polypeptides may display a preferred subcellular localization, typically one or more of nuclear, cytoplasmic, chloroplastic, or mitochondrial. The task of protein subcellular localisation prediction is important and well studied. Knowing a protein's localisation helps elucidate its function. Experimental methods for protein localization range from immunolocalization to tagging of proteins using green fluorescent protein (GFP) or beta-glucuronidase (GUS). Such methods are accurate although labour-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, LocTree, Predotar, LipoP, MITOPROT, PATS, PTS1, SignalP, TMHMM, and others.

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any IAA2-like-encoding nucleic acid or IAA2-like polypeptide as defined herein having at least 80% overall sequence identity to the amino acid sequence represented by SEQ ID NO: 2.

Examples of nucleic acids encoding IAA2-like polypeptides are given in Table A1 of the Examples section herein. Such nucleic acids encoding polypeptides having at least 80% sequence identity to SEQ ID NO: 2 are useful in performing the methods of the invention. The amino acid sequences given in Table A1 of the Examples section are example sequences of orthologues and paralogues of the IAA2-like polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A1 of the Examples section) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against rice sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the disclosure. Examples of such variants include nucleic acids encoding homologues and derivatives of any one of the amino acid sequences given in Table A1 of the Examples section, the terms "homologue" and "derivative" being as defined herein. Also useful in the methods of the disclosure are nucleic acids encoding homologues and derivatives of orthologues or paralogues of any one of the amino acid sequences given in Table A1 of the Examples section. Homologues and derivatives useful in the methods of the present disclosure have substantially the same biological and functional activity as the unmodified protein from which they are derived. Further variants useful in practising the methods of the disclosure are variants in which codon usage is optimised or in which miRNA target sites are removed.

Further nucleic acid variants useful in practising the methods of the disclosure include portions of nucleic acids encoding IAA-like polypeptides, nucleic acids hybridising to nucleic acids encoding IAA-like polypeptides, splice variants of nucleic acids encoding IAA-like polypeptides, allelic variants of nucleic acids encoding IAA-like polypeptides and variants of nucleic acids encoding IAA-like polypeptides obtained by gene shuffling. The terms hybridising sequence, splice variant, allelic variant and gene shuffling are as described herein.

Nucleic acids encoding IAA-like polypeptides need not be full-length nucleic acids, since performance of the methods of the disclosure does not rely on the use of full-length nucleic acid sequences. According to the presentdisclosure, there is provided a method for increasing seed yield in plants, comprising introducing and expressing in a plant a portion of any one of the nucleic acid sequences given in Table A1 of the Examples section, or a portion of a nucleic acid encoding an orthologue, paralogue or homologue of any of the amino acid sequences given in Table A1of the Examples section.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods, encode an IAA2-like polypeptide as defined herein, and have substantially the same biological activity as the amino acid sequences given in Table A1 of the Examples section. Preferably, the portion is a portion of any one of the nucleic acids given in Table A1 of the Examples section, or is a portion of a nucleic acid encoding an orthologue or paralogue of any one of the amino acid sequences given in Table A1 of the Examples section. Preferably the portion is at least 100, 200, 300, 400, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700 consecutive nucleotides in length, the consecutive nucleotides being of any one of the nucleic acid sequences given in Table A1 of the Examples section, or of a nucleic acid encoding an orthologue or paralogue of any one of the amino acid sequences given in Table A1 of the Examples section. Most preferably the portion is a portion of the nucleic acid of SEQ ID NO: 1. Preferably, the portion encodes a fragment of an amino acid sequence comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

Another nucleic acid variant useful in the methods is a nucleic acid capable of hybridising, under reduced stringency conditions, preferably under stringent conditions, with a nucleic acid encoding an IAA-like polypeptide as defined herein, or with a portion as defined herein.

There is provided a method for increasing seed yield in plants, comprising introducing and expressing in a plant a nucleic acid capable of hybridising to any one of the nucleic acids given in Table A1 of the Examples section, or comprising introducing and expressing in a plant a nucleic acid capable of hybridising to a nucleic acid encoding an orthologue, paralogue or homologue of any of the nucleic acid sequences given in Table A1of the Examples section.

Hybridising sequences useful in the methods encode an IAA2-like polypeptide as defined herein, having substantially the same biological activity as the amino acid sequences given in Table A1 of the Examples section. Preferably, the hybridising sequence is capable of hybridising to the complement of any one of the nucleic acids given in Table A1 of the Examples section, or to a portion of any of these sequences, a portion being as defined above, or the hybridising sequence is capable of hybridising to the complement of a nucleic acid encoding an orthologue or paralogue of any one of the amino acid sequences given in Table A1 of the Examples section. Most preferably, the hybridising sequence is capable of hybridising to the complement of a nucleic acid as represented by SEQ ID NO: 1 or to a portion thereof.

Preferably, the hybridising sequence or its complementary sequence encodes a polypeptide with an amino acid sequence comprising an AUX/IAA domain (PFAM accession number PF2309, InterPro entry IPR003311) and the protein motif: Motif 1, SEQ ID NO: 22: (K/N)(I/M/L)F(S/Y)(Q/G)L.

Another nucleic acid variant useful in the methods is a splice variant encoding an IAA-like polypeptide as defined hereinabove, a splice variant being as defined herein.

According to the present disclosure, there is provided a method for increasing seed yield in plants, comprising introducing and expressing in a plant a splice variant of any one of the nucleic acid sequences given in Table A1 or Table A2 of the Examples section, or a splice variant of a nucleic acid encoding an orthologue, paralogue or homologue of any of the amino acid sequences given in Table A1 of the Examples section.

Concerning IAA2-like polypeptides, preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Another nucleic acid variant useful in performing the methods is an allelic variant of a nucleic acid encoding an IAA-like polypeptide as defined hereinabove, an allelic variant being as defined herein.

There is provided a method for increasing seed yield, comprising introducing and expressing in a plant an allelic variant of any one of the nucleic acids given in Table A1of the Examples section, or comprising introducing and expressing in a plant an allelic variant of a nucleic acid encoding an orthologue, paralogue or homologue of any of the amino acid sequences given in Table A1of the Examples section.

The polypeptides encoded by allelic variants useful in the methods have substantially the same biological activity as the IAA2-like polypeptide of SEQ ID NO: 2 and any of the amino acids depicted in Table A1 of the Examples section. Allelic variants exist in nature, and encompassed within the methods is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Gene shuffling or directed evolution may also be used to generate variants of nucleic acids encoding IAA-like polypeptides as defined above; the term "gene shuffling" being as defined herein.

There is provided a method for increasing seed yield, comprising introducing and expressing in a plant a variant of any one of the nucleic acid sequences given in Table A1 of the Examples section, or comprising introducing and expressing in a plant a variant of a nucleic acid encoding an orthologue, paralogue or homologue of any of the amino acid sequences given in Table A1 of the Examples section, which variant nucleic acid is obtained by gene shuffling.

Described herein are IAA2-like polypeptides in which the amino acid sequence encoded by the variant nucleic acid obtained by gene shuffling comprises an AUX/IAA domain (PFAM accession number PF2309, InterPro entry IPR003311) and the protein motif: Motif 1, SEQ ID NO: 22: (K/N)(I/M/L)F(S/Y)(Q/G)L.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding IAA2-like polypeptides may be derived from any natural or artificial source. The nucleic acid may be modified from its native form in composition and/or genomic environment through deliberate human manipulation. Preferably the IAA2-like polypeptide-encoding nucleic acid is from a plant, further preferably from a monocotyledonous plant, more preferably from the family Poaceae, most preferably the nucleic acid is from *Oryza sativa.*

Performance of the methods of the invention gives plants having increased seed yield. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased seed yield relative to the seed yield of control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others.

Taking rice as an example, a yield increase may manifest itself as an increase in one or more of the following: number of plants per square meter, number of panicles per plant, panicle length, number of spikelets per panicle, number of flowers (florets) per panicle, increase in the seed filling rate (which is the number of filled seeds divided by the total number of seeds and multiplied by 100), increase in thousand kernel weight, among others. In rice, submergence tolerance may also result in increased yield.

The present invention provides a method for increasing seed yield of plants, relative to control plants, which method comprises introduction and overexpression in a plant of a nucleic acid encoding an IAA-like polypeptide as defined herein.

Since the transgenic plants according to the present invention have increased seed yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as speed of germination, early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

Performance of the methods of the disclosure gives plants having an increased growth rate relative to control plants. Therefore, according to the present disclosure, there is provided a method for increasing the growth rate of plants, which method comprises overexpression in a plant of a nucleic acid encoding an IAA-like polypeptide as defined herein.

An increase in seed yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi, nematodes and insects. The term "non-stress" conditions as used herein are those environmental conditions that allow optimal growth of plants. Persons skilled in the art are aware of normal soil conditions and climatic conditions for a given location. The term non-stress conditions as used herein, encompasses the occasional or everyday mild stresses to which a plant is exposed, as defined herein, but does not encompass severe stresses.

In particular, the methods of the present invention may be performed under conditions of drought, cold or salt stress, to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 97%, 95%, 92%, 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under non-stress conditions or under mild drought conditions increased seed yield relative to control plants grown under comparable conditions. Therefore, according to the present invention, there is provided a method for increasing seed yield in plants grown under non-stress conditions or under mild drought conditions, which method comprises introduction and overexpression in a plant of a nucleic acid encoding an IAA2-like polypeptide.

Performance of the methods of the invention gives plants grown under conditions of nutrient deficiency, particularly under conditions of nitrogen deficiency, increased yield relative to control plants grown under comparable conditions. Because of the strong influence of nutrition utilization efficiency on plant yield and product quality, a huge amount of fertilizer is poured onto fields to optimize plant growth and quality. Productivity of plants ordinarily is limited by three primary nutrients, phosphorous, potassium and nitrogen, which is usually the rate-limiting element in plant growth of these three. Therefore the major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor for crop plant growth and production (Frink et al. (1999) Proc Natl Acad Sci USA 96(4): 1175-1180), and has as well a major impact on protein accumulation and amino acid composition. Therefore, of great interest are crop plants with increased yield-related traits, when grown under nitrogen-limiting conditions. Thus, according to the present invention, there is provided a method for increasing seed yield in plants grown under conditions of nutrient deficiency, which method comprises introduction and overexpression in a plant of a nucleic acid encoding an IAA-like polypeptide. Nutrient deficiency may result from a lack of nutrients such as nitrogen, phosphates and other phosphorous-containing compounds, potassium, calcium, magnesium, manganese, iron and boron, amongst others.

Described herein is performance of the methods of the invention to give plants grown under conditions of salt stress increased seed yield relative to control plants grown under comparable conditions. Therefore, there is provided a method for increasing seed yield in plants grown under conditions of salt stress, which method comprises introduction and overexpression in a plant of a nucleic acid encoding an IAA-like polypeptide. The term salt stress is not restricted to common salt (NaCl), but may be any one or more of: NaCl, KCl, LiCl, $MgCl_2$, $CaCl_2$, amongst others.

The present invention encompasses plants or parts thereof (including seeds) obtainable by the methods according to the present invention. The plants or parts thereof comprise a nucleic acid transgene encoding an IAA-like polypeptide as defined above, which nucleic acid is operably linked to a GOS2 promoter.

The invention also provides genetic constructs and vectors to facilitate introduction and/or overexpression in plants of nucleic acids encoding IAA-like polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(a) a nucleic acid encoding an IAA-like polypeptide as defined above;
(b) one or more control sequences capable of driving expression of the nucleic acid sequence of (a), wherein one of said control sequences is a GOS2 promoter; and optionally
(c) a transcription termination sequence.

The nucleic acid encoding an IAA-like polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a GOS2 promoter).

Advantageously, any type of promoter, whether natural or synthetic, may be used to drive expression of the nucleic acid sequence, but preferably the promoter is of plant origin. A constitutive promoter is particularly useful in the methods. Preferably the constitutive promoter is a ubiquitous constitutive promoter of medium strength. See the "Definitions" section herein for definitions of the various promoter types.

It should be clear that the applicability of the present invention is not restricted to the IAA2-like polypeptide-encoding nucleic acid represented by SEQ ID NO: 1, nor is the applicability of the invention restricted to expression of an IAA2-like polypeptide-encoding nucleic acid when driven by a constitutive promoter.

The constitutive promoter is preferably a medium strength promoter, more preferably selected from a plant derived promoter, such as a GOS2 promoter, more preferably the promoter is a GOS2 promoter from rice. Further preferably the constitutive promoter is represented by a nucleic acid sequence substantially similar to SEQ ID NO: 26, most preferably the constitutive promoter is as represented by SEQ ID NO: 26. See the "Definitions" section herein for further examples of constitutive promoters.

Optionally, one or more terminator sequences may be used in the construct introduced into a plant. Preferably, the construct comprises an expression cassette comprising a GOS2 promoter, substantially similar to SEQ ID NO: 26, and the nucleic acid encoding the IAA2-like polypeptide.

Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

Also described herein is a method for the production of transgenic plants having enhanced yield-related traits relative to control plants, comprising introduction and expression in a plant of any nucleic acid encoding an IAA-like polypeptide as defined hereinabove.

More specifically, described herein is a method for the production of transgenic plants having increased seed yield, which method comprises:

(i) introducing and expressing in a plant or plant cell a nucleic acid encoding an IAA-like polypeptide; and
(ii) cultivating the plant cell under conditions promoting plant growth and development.

The nucleic acid of (i) may be any of the nucleic acids capable of encoding an IAA-like polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present disclosure clearly extends to any plant cell or plant produced by any of the disclosure methods described herein, and to all plant parts and propagules thereof. The present disclosure extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the disclosure.

The disclosure also includes host cells containing an isolated nucleic acid encoding an IAA-like polypeptide as defined hereinabove. Preferred host cells are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, linseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum, emmer, spelt, secale, einkorn, teff, milo and oats.

The invention also extends to harvestable parts of a plant such as, but not limited to seeds, leaves, fruits, flowers, stems, roots, rhizomes, tubers and bulbs, which harvestable parts comprise a recombinant nucleic acid encoding an IAA-like polypeptide. The disclosure furthermore relates to products derived, preferably directly derived, from a harvestable part of such a plant, such as dry pellets or powders, oil, fat and fatty acids, starch or proteins.

According to the invention, the modulated expression is increased expression. Methods for increasing expression of nucleic acids or genes, or gene products, are well documented in the art and examples are provided in the definitions section.

A preferred method for increasing expression of a nucleic acid encoding an IAA-like polypeptide is by introducing and expressing in a plant a nucleic acid encoding an IAA-like polypeptide; however the effects of performing the method, i.e. increased seed yield may also be achieved using other well known techniques, including but not limited to T-DNA activation tagging, TILLING, homologous recombination. A description of these techniques is provided in the definitions section.

The present invention also encompasses use of nucleic acids encoding IAA-like polypeptides as described herein in enhancing seed yield in plants.

Nucleic acids encoding IAA-like polypeptides may find use in breeding programmes in which a DNA marker is identified which may be genetically linked to a gene encoding an IAA-like polypeptide. The nucleic acids/genes, or the IAA-like polypeptides themselves may be used to define a molecular marker. This DNA or protein marker may then be used in breeding programmes to select plants having enhanced yield-related traits as defined hereinabove in the methods of the invention.

Allelic variants of a nucleic acid/gene encoding IAA-like polypeptides may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding IAA-like polypeptides may also be used as probes for genetically and physically mapping the genes that they are a part of, and as markers for traits linked to those genes. Such information may be useful in plant breeding in order to develop lines with desired phenotypes. Such use of nucleic acids encoding IAA-like polypeptides requires only a nucleic acid sequence of at least 15 nucleotides in length. The nucleic acids encoding IAA-like polypeptides may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the IAA-like-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid encoding IAA-like polypeptides in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

The nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased seed yield, as described hereinbefore. These traits may also be combined with other economically advantageous traits, such as further yield-enhancing traits, tolerance to other abiotic and biotic stresses, traits modifying various architectural features and/or biochemical and/or physiological features.

## Description of figures

The present invention will now be described with reference to the following figures in which:

**Figure 1** represents a multiple alignment of IAA2-like polypeptides.
**Figure 2** represents the binary vector used for increased expression in *Oryza sativa* of an IAA2-like-encoding nucleic acid under the control of a rice GOS2 promoter (pGOS2).

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

*Example 1: Identification of sequences related to the nucleic acid sequence used in the methods of the invention*

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

<u>1.1. IAA2-like polypeptides</u>

Table A1 provides a list of nucleic acid sequences related to the IAA2-like nucleic acid sequence useful in the methods of the present invention.

**Table A1:** Examples of IAA2-like polypeptides:

| Name | Nulcleic add SEQ ID NO: | Polypeptide SEQ ID NO: |
|---|---|---|
| O.sativa_Os01g0741900#1 | 1 | 2 |
| O.sativa_Os05g0523300#1 | 3 | 4 |
| corn IAA6likeACF83856.1 | 5 | 6 |
| corn_EU965307_IAA6_ACG37425 | 7 | 8 |
| H.vulgare-TC162609#1 | 9 | 10 |
| OsIBCD003255\|319\|AUX-IAA indica | 11 | 12 |
| T.aestivum_TC289502#1 | 13 | 14 |
| T.aestivum_TC297212#1 | 15 | 16 |
| T.aestivum-TC350242#1 | 17 | 18 |
| Z.mays_TC410949#1 | 19 | 20 |

In some instances, related sequences have tentatively been assembled and publicly disclosed by research institutions, such as The Institute for Genomic Research (TIGR; beginning with TA). The Eukaryotic Gene Orthologs (EGO) database may be used to identify such related sequences, either by keyword search or by using the BLAST algorithm with the nucleic acid sequence or polypeptide sequence of interest. On other instances, special nucleic acid sequence databases have been created for particular organisms, such as by the Joint Genome Institute. Further, access to proprietary databases, has allowed the identification of novel nucleic acid and polypeptide sequences.

*Example 2: Alignment of sequences related to the polypeptide sequences used in the methods of the invention*

2.1. IAA2-like polypeptides

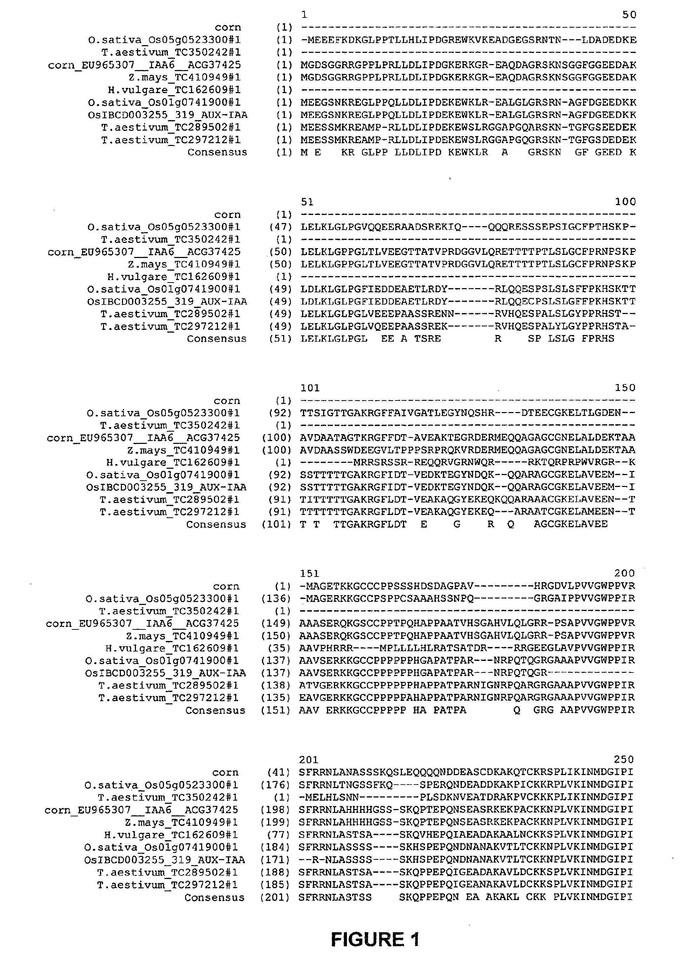

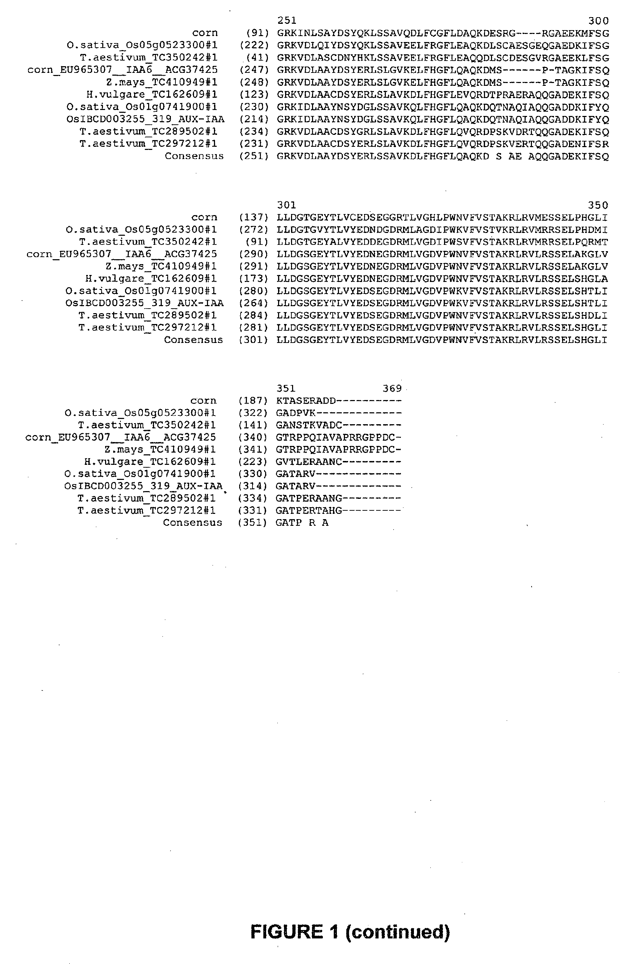

Alignment of polypeptide sequences was performed using the AlignX programme from the Vector NTI (Invitrogen), which is based on the ClustalW 2.0 algorithm for progressive alignment (Thompson et al. (1997) Nucleic Acids Res 25:4876-4882; Chenna et al. (2003). Nucleic Acids Res 31:3497-3500); Alingment was performed with standard settings: gap opening penalty 10, gap extension penalty: 0.2. Minor manual editing was done to further optimise the alignment. The IAA2-like polypeptides are aligned in Figure 1.
Highly conserved amino acid residues are indicated in the consensus sequence.

*Example 3: Calculation of global percentage identity between polypeptide sequences useful in performing the methods of the invention*

3.1. IAA2-like polypeptides

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix.
Parameters that may be used in the comparison:

| | |
|---|---|
| Scoring matrix: | Blosum62 |
| First Gap: | 12 |
| Extending gap: | 2 |

*Example 4: Identification of domains comprised in polypeptide sequences useful in performing the methods of the invention*

4.1. IAA2-like polypeptides

The presence of conserved protein domains in SEQ ID NO: 2 was determined by searching the pfam database. Pfam is a large collection of multiple sequence alignments and hidden Markov models covering many common protein domains and families. Pfam is hosted at the Sanger Institute server in the United Kingdom.
The results of the search of the Pfam with the query sequence as represented by SEQ ID NO: 2 are presented in Table C1.

**Table C1:** Pfam search results (major accession numbers) of the polypeptide sequence as represented by SEQ ID NO: 2.

| | Description | Entry type | Sequence (amino acid coordinates of the domain in the query sequence) | | E-value | Alignment mode |
|---|---|---|---|---|---|---|
| | | | Start | End | | |
| **Pfam-A** | AUX/IAA family | Family | 49 | 335 | 3E-13 | ls |

The Alignment mode used for PF2309 is the so called "ls". Parameters used in the model are given in Table C2.

**Table C2:**

| ls model: hmmbuild -F HMM_ls SEED hmmcalibrate --cpu 1 --seed 0 HUM_ls | | |
|---|---|---|
| **Parameter** | ls | |
| | **Sequence** | **Domain** |
| Gathering cut-off | -83 | -83 |
| Trusted cut-off | -82 | -82 |
| Noise cut-off | -83.5 | -83.5 |

*Example 5: Topology prediction of the polypeptide sequences useful in performing the methods of the invention*

5.1. IAA2-like polypeptides

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters are selected, such as organism group (non-plant or plant), cutoff sets (none, predefined set of cutoffs, or user-specified set of cutoffs), and the calculation of prediction of cleavage sites (yes or no).

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;
- TMHMM, hosted on the server of the Technical University of Denmark
- PSORT (URL: psort.org)
- PLOC (Park and Kanehisa, Bioinformatics, 19, 1656-1663, 2003).

*Example 6: Cloning of the nucleic acid sequence used in the methods of the invention and rice transformation*

6.1. IAA2-like polypeptides

The nucleic acid having the sequence of SEQ ID NO: 1 was isolated using routine molecular biology techniques from rice and cloned into an entry vector of the Gateway technology (Invitrogen).

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 26) for constitutive specific expression was located upstream of this Gateway cassette.

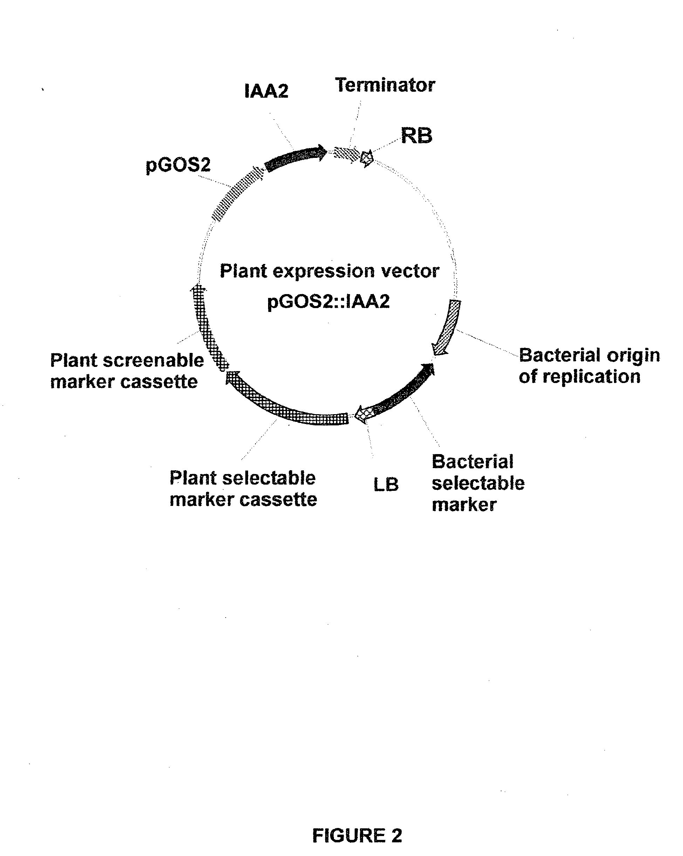

After the LR recombination step, the resulting expression vector pGOS2::IAA2-like (Figure 2) was transformed into *Agrobacterium* strain LBA4044 according to methods well known in the art.

*Example 8: Plant transformation of other crops*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl2, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Example 8: Transformation of other crops*

*Corn transformation*

Transformation of maize *(Zea mays)* is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25°C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5-10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa *(Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings are transplanted into pots and grown in a greenhouse. T1 seeds are produced from plants that exhibit tolerance to the selection agent and that contain a single copy of the T-DNA insert.

*Cotton transformation*

Cotton is transformed using *Agrobacterium tumefaciens* according to the method described in US 5,159,135. Cotton seeds are surface sterilised in 3% sodium hypochlorite solution during 20 minutes and washed in distilled water with 500 μg/ml cefotaxime. The seeds are then transferred to SH-medium with 50μg/ml benomyl for germination. Hypocotyls of 4 to 6 days old seedlings are removed, cut into 0.5 cm pieces and are placed on 0.8% agar. An *Agrobacterium* suspension (approx. 108 cells per ml, diluted from an overnight culture transformed with the gene of interest and suitable selection markers) is used for inoculation of the hypocotyl explants. After 3 days at room temperature and lighting, the tissues are transferred to a solid medium (1.6 g/l Gelrite) with Murashige and Skoog salts with B5 vitamins (Gamborg et al., Exp. Cell Res. 50:151-158 (1968)), 0.1 mg/l 2,4-D, 0.1 mg/l 6-furfurylaminopurine and 750 μg/ml MgCL2, and with 50 to 100 μg/ml cefotaxime and 400-500 μg/ml carbenicillin to kill residual bacteria. Individual cell lines are isolated after two to three months (with subcultures every four to six weeks) and are further cultivated on selective medium for tissue amplification (30°C, 16 hr photoperiod). Transformed tissues are subsequently further cultivated on non-selective medium during 2 to 3 months to give rise to somatic embryos. Healthy looking embryos of at least 4 mm length are transferred to tubes with SH medium in fine vermiculite, supplemented with 0.1 mg/l indole acetic acid, 6 furfurylaminopurine and gibberellic acid. The embryos are cultivated at 30°C with a photoperiod of 16 hrs, and plantlets at the 2 to 3 leaf stage are transferred to pots with vermiculite and nutrients. The plants are hardened and subsequently moved to the greenhouse for further cultivation.

*Example 9: Phenotypic evaluation procedure*

9.1. IAA2-like polypeptides

9.1.1. Evaluation setup

Approximately 35 independent T0 rice transformants were generated. The primary transformants were transferred from a tissue culture chamber to a greenhouse for growing and harvest of T1 seed. Events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions were watered at regular intervals to ensure that water and nutrients were not limiting and to satisfy plant needs to complete growth and development. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

*Salt stress screen*

Plants are grown on a substrate made of coco fibers and argex (3 to 1 ratio). A normal nutrient solution is used during the first two weeks after transplanting the plantlets in the greenhouse. After the first two weeks, 25 mM of salt (NaCl) is added to the nutrient solution, until the plants are harvested. Seed-related parameters are then measured.

9.1.2. Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

9.1.3. Parameters measured

*Biomass-related parameter measurement*

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time point from different angles and was converted to a physical surface value expressed in square mm by calibration. The results described below are for plants three weeks post-germination.

*Seed-related parameter measurements*

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

*Examples 10: Results of the phenotypic evaluation of the transgenic plants*

<u>10.1. IAA2-like polypeptides</u>

The results of the evaluation of transgenic rice plants in the T2 generation and expressing a nucleic acid comprising the longest Open Reading Frame in SEQ ID NO: 1 under non-stress conditions are presented below. See previous Examples for details on the generations of the transgenic plants.

The results of the evaluation of transgenic rice plants under non-stress conditions are presented below (Table E1). An increase of at least 5 % was observed for seed fill rate (fill rate) and harvest index (harvest index), and of 2.5% for thousand kernel weight (TKW).

**Table E1**

| Yield-related trait | Percentage increase in transgenic plants compared to control plants |
|---|---|
| Fill rate | 16.3 |
| TKW | 3 |
| Harvest index | 11.1 |

## Claims

1. A method for increasing seed yield in plants relative to control plants, comprising introducing and over expressing in a plant a nucleic acid encoding an IAA2-like polypeptide, wherein said IAA2-like polypeptide is represented by SEQ ID NO: 2 or is a polypeptide having at least 80% overall sequence identity to the amino acid sequence represented by SEQ ID NO: 2.

2. Method according to claim 1, wherein said nucleic acid is represented by SEQ ID NO: 1 and encodes the IAA2-like polypeptide of SEQ ID NO: 2.

3. Method according to claim 1 or 2, wherein said increased seed yield is obtained under non-stress conditions.

4. Method according to any one of claims 1 to 3, wherein said nucleic acid is operably linked to a constitutive promoter, preferably to a GOS2 promoter, most preferably to a GOS2 promoter from rice.

5. Method according to any one of claims 1 to 4, wherein said nucleic acid encoding an IAA2-like polypeptide is of plant origin, preferably from a monocotyledonous plant, further preferably from the family Poaceae, more preferably from the genus Oryza, most preferably from *Oryza sativa.*

6. Plant or part thereof, including seeds, obtainable by a method according to any one of claims 1 to 5, wherein said plant or part thereof comprises a recombinant nucleic acid encoding an IAA2-like polypeptide as defined in claim 1 operably linked to a GOS2 promoter.

7. Construct comprising:

   (i) nucleic acid encoding an IAA2-like polypeptide as defined in claim 1;
   (ii) one or more control sequences capable of driving expression of the nucleic acid sequence of (i), wherein one of said control sequences is a GOS2 promoter, preferably a GOS2 promoter from rice; and optionally
   (iii) a transcription termination sequence.

8. Plant, plant part or plant cell comprising a construct according to claim 7.

9. Transgenic plant having increased seed yield, relative to control plants, resulting from overexpression of a nucleic acid encoding an IAA2-like polypeptide as defined in claim 1, or a transgenic plant cell derived from said transgenic plant, wherein said nucleic acid is operably linked to a GOS2 promoter.

10. Transgenic plant according to claim 6, 8 or 9, or a transgenic plant cell derived thereof, wherein said plant is a crop plant or a monocot or a cereal, such as rice, maize, wheat, barley, millet, rye, triticale, sorghum, emmer, spelt, secale, einkorn, teff, milo and oats.

11. Harvestable parts of a plant according to claim 10, wherein said harvestable parts are seeds and comprise the nucleic acid as defined in claim 9.

12. Use of a nucleic acid encoding an IAA2-like polypeptide represented by SEQ ID NO: 2 or a polypeptide having at least 80% overall sequence identity to the amino acid sequence represented by SEQ ID NO: 2 in increasing seed yield in plants, relative to control plants, or use of a construct according to claim 7 in a method for making plants having increased seed yield relative to control plants.

## Patentansprüche

1. Verfahren zur Steigerung des Samenertrags in Pflanzen im Vergleich zu Kontrollpflanzen, bei dem man in eine Pflanze eine Nukleinsäure, die für ein IAA2-ähnliches Polypeptid codiert, einführt und darin überexprimiert, wobei das IAA2-ähnliche Polypeptid durch SEQ ID NO: 2 dargestellt wird oder ein Polypeptid mit mindestens 80% Gesamtsequenzidentität zu der durch SEQ ID NO: 2 dargestellten Aminosäuresequenz ist.

2. Verfahren nach Anspruch 1, wobei die Nukleinsäure durch SEQ ID NO: 1 dargestellt ist und für das IAA2-ähnliche Polypeptid der SEQ ID NO: 2 codiert.

3. Verfahren nach Anspruch 1 oder 2, wobei der gesteigerte Samenertrag unter Nichtstressbedingungen erhalten wird.

4. Verfahren nach einem der Ansprüche 1 bis 3, wobei die Nukleinsäure funktionsfähig mit einem konstitutiven Promotor, vorzugsweise einem GOS2-Promotor, am stärksten bevorzugt einem GOS2-Promotor aus Reis, verbunden ist.

5. Verfahren nach einem der Ansprüche 1 bis 4, wobei die Nukleinsäure, die für ein IAA2-ähnliches Polypeptid codiert, aus einer Pflanze, vorzugsweise aus einer monokotylen Pflanze, weiter bevorzugt aus der Familie Poaceae, stärker bevorzugt aus der Gattung Oryza, am stärksten bevorzugt aus *Oryza sativa,* stammt.

6. Pflanze oder Teil davon, einschließlich Samen, die durch ein Verfahren nach einem der Ansprüche 1 bis 5 erhältlich sind, wobei die Pflanze oder der Teil davon eine rekombinante Nukleinsäure umfasst, die für ein wie in Anspruch 1 definiertes IAA2-ähnliches Polypeptid, das funktionsfähig mit einem GOS2-Promotor verbunden ist, codiert.

7. Konstrukt, umfassend:

(i) eine Nukleinsäure, die für ein wie in Anspruch 1 definiertes IAA2-ähnliches Polypeptid codiert;
(ii) eine oder mehrere Kontrollsequenzen, die die Expression der Nukleinsäuresequenz unter (i) steuern können, wobei eine der Kontrollsequenzen ein GOS2-Promotor, vorzugsweise ein GOS2-Promotor aus Reis, ist; und gegebenenfalls
(iii) eine Transkriptionsterminationssequenz.

8. Pflanze, Pflanzenteil oder Pflanzenzelle, die/der ein Konstrukt nach Anspruch 7 umfasst.

9. Transgene Pflanze mit gesteigertem Samenertrag im Vergleich zu Kontrollpflanzen, der sich aus einer Überexpression einer Nukleinsäure, die für ein wie in Anspruch 1 definiertes IAA2-ähnliches Polypeptid codiert, ergibt, oder transgene Pflanzenzelle, die aus der transgenen Pflanze stammt, wobei die Nukleinsäure funktionsfähig mit einem GOS2-Promotor verbunden ist.

10. Transgene Pflanze nach Anspruch 6, 8 oder 9 oder eine daraus stammende transgene Pflanzenzelle, wobei die Pflanze eine Kulturpflanze oder eine Monokotyle oder ein Getreide, wie Reis, Mais, Weizen, Gerste, Hirse, Roggen, Triticale, Sorghum, Emmer, Dinkel, Secale, Einkorn, Zwerghirse, Mohrenhirse und Hafer ist.

11. Erntbare Teile einer Pflanze nach Anspruch 10, wobei die erntbaren Teile Samen sind und die wie in Anspruch 9 definierte Nukleinsäure umfassen.

12. Verwendung einer Nukleinsäure, die für ein durch SEQ ID NO: 2 dargestelltes IAA2-ähnliches Polypeptid oder ein Polypeptid mit mindestens 80% Gesamtsequenzidentität zu der durch SEQ ID NO: 2 dargestellten Aminosäuresequenz codiert, zur Steigerung des Samenertrags in Pflanzen im Vergleich zu Kontrollpflanzen, oder die Verwendung eines Konstrukts nach Anspruch 7 in einem Verfahren zur Herstellung von Pflanzen mit gesteigertem Samenertrag im Vergleich zu Kontrollpflanzen.

## Revendications

1. Procédé pour augmenter le rendement en graines dans des plantes par comparaison avec des plantes témoins, comprenant l'introduction et la surexpression, dans une plante, d'un acide nucléique codant pour un polypeptide de type IAA2, ledit polypeptide de type IAA2 étant représenté par SEQ ID NO:2 ou étant un polypeptide ayant une identité globale de séquence d'au moins 80 % avec la séquence d'acides aminés représentée par SEQ ID NO:2.

2. Procédé selon la revendication 1, dans lequel ledit acide nucléique est représenté par SEQ ID NO:1 et code pour le polypeptide de type IAA2 SEQ ID NO:2.

3. Procédé selon la revendication 1 ou 2, dans lequel ledit rendement en graines accru est obtenu dans des conditions sans stress.

4. Procédé selon l'une quelconque des revendications 1 à 3, dans lequel ledit acide nucléique est fonctionnellement lié à un promoteur constitutif, de préférence un promoteur GOS2, tout spécialement un promoteur GOS2 du riz.

**5.** Procédé selon l'une quelconque des revendications 1 à 4, dans lequel ledit acide nucléique codant pour un polypeptide de type IAA2 est d'origine végétale, provient de préférence d'une plante monocotylédone, plus particulièrement de la famille des Poaceae, d'une manière plus préférée du genre Oryza, tout spécialement d'*Oryza sativa.*

**6.** Plante ou partie de plante, y compris les graines, pouvant être obtenue par un procédé selon l'une quelconque des revendications 1 à 5, ladite plante ou partie de plante comprenant un acide nucléique recombinant codant pour un polypeptide de type IAA2 tel que défini dans la revendication 1, fonctionnellement lié à un promoteur GOS2.

**7.** Construction comprenant :

(i) un acide nucléique codant pour un polypeptide de type IAA2 tel que défini dans la revendication 1 ;
(ii) une ou plusieurs séquences de contrôle, capables de commander l'expression de la séquence d'acide nucléique de (i), l'une desdites séquences de contrôle étant un promoteur GOS2, de préférence un promoteur GOS2 du riz ; et, en option
(iii) une séquence terminatrice de transcription.

**8.** Plante, partie de plante ou cellule végétale comprenant une construction selon la revendication 7.

**9.** Plante transgénique ayant un rendement en graines accru par rapport à des plantes témoins, résultant de la surexpression d'un acide nucléique codant pour un polypeptide de type IAA2 tel que défini dans la revendication 1, ou cellule végétale transgénique dérivée de ladite plante transgénique, ledit acide nucléique étant fonctionnellement lié à un promoteur GOS2.

**10.** Plante transgénique selon la revendication 6, 8 ou 9, ou cellule végétale transgénique qui en dérive, ladite plante étant une plante de culture ou une monocotylédone ou une céréale telle que le riz, le maïs, le blé, l'orge, le millet, le seigle, le triticale, le sorgo, l'amidonnier, l'épeautre, le sécale, l'engrain, le teff, le milo et l'avoine.

**11.** Parties récoltables d'une plante selon la revendication 10, lesdites plantes récoltables étant des graines et comprenant l'acide nucléique tel que défini dans la revendication 9.

**12.** Utilisation d'un acide nucléique codant pour un polypeptide de type IAA2 représenté par SEQ ID NO:2 ou pour un polypeptide ayant une identité globale de séquence d'au moins 80 % avec la séquence d'acides aminés représentée par SEQ ID NO:2, pour augmenter le rendement en graines de plantes, par rapport à des plantes témoins, ou utilisation d'une construction selon la revendication 7 dans un procédé de fabrication de plantes ayant un rendement en graines accru par rapport à des plantes témoins.

```
                                     1                                                50
                     corn      (1)   --------------------------------------------------
   O.sativa_Os05g0523300#1     (1)   -MEEEFKDKGLPPTLLHLIPDGREWKVKEADGEGSRNTN---LDADEDKE
     T.aestivum_TC350242#1     (1)   --------------------------------------------------
 corn_EU965307__IAA6__ACG37425 (1)   MGDSGGRRGPPLPRLLDLIPDGKERKGR-EAQDAGRSKNSGGFGGEEDAK
        Z.mays_TC410949#1      (1)   MGDSGGRRGPPLPRLLDLIPDGKERKGR-EAQDAGRSKNSGGFGGEEDAK
      H.vulgare_TC162609#1     (1)   --------------------------------------------------
   O.sativa_Os01g0741900#1     (1)   MEEGSNKREGLPPQLLDLIPDEKEWKLR-EALGLGRSRN-AGFDGEEDKK
   OsIBCD003255_319_AUX-IAA    (1)   MEEGSNKREGLPPQLLDLIPDEKEWKLR-EALGLGRSRN-AGFDGEEDKK
     T.aestivum_TC289502#1     (1)   MEESSMKREAMP-RLLDLIPDEKEWSLRGGAPGQARSKN-TGFGSEEDEK
     T.aestivum_TC297212#1     (1)   MEESSMKREAMP-RLLDLIPDEKEWSLRGGAPGQGRSKN-TGFGSDEDEK
                Consensus      (1)   M E   KR GLPP LLDLIPD KEWKLR  A   GRSKN  GF GEED K

                                     51                                              100
                     corn      (1)   --------------------------------------------------
   O.sativa_Os05g0523300#1    (47)   LELKLGLPGVQQEERAADSREKIQ----QQQRESSSEPSIGCFPTHSKP-
     T.aestivum_TC350242#1     (1)   --------------------------------------------------
 corn_EU965307__IAA6__ACG37425 (50)  LELKLGPPGLTLVEEGTTATVPRDGGVLQRETTTTPTLSLGCFPRNPSKP
        Z.mays_TC410949#1     (50)   LELKLGPPGLTLVEEGTTATVPRDGGVLQRETTTTPTLSLGCFPRNPSKP
      H.vulgare_TC162609#1     (1)   --------------------------------------------------
   O.sativa_Os01g0741900#1    (49)   LDLKLGLPGFIEDDEAETLRDY-------RLQQESPSLSLSFFPKHSKTT
   OsIBCD003255_319_AUX-IAA   (49)   LDLKLGLPGFIEDDEAETLRDY-------RLQQECPSLSLGFFPKHSKTT
     T.aestivum_TC289502#1    (49)   LELKLGLPGLVEEEPAASSRENN------RVHQESPALSLGYPPRHST--
     T.aestivum_TC297212#1    (49)   LELKLGLPGLVQEEPAASSREK-------RVHQESPALYLGYPPRHSTA-
                Consensus     (51)   LELKLGLPGL  EE A TSRE        R    SP LSLG FPRHS

                                     101                                             150
                     corn      (1)   --------------------------------------------------
   O.sativa_Os05g0523300#1    (92)   TTSIGTTGAKRGFFAIVGATLEGYNQSHR----DTEECGKELTLGDEN--
     T.aestivum_TC350242#1     (1)   --------------------------------------------------
 corn_EU965307__IAA6__ACG37425 (100) AVDAATAGTKRGFFDT-AVEAKTEGRDERMEQQAGAGCGNELALDEKTAA
        Z.mays_TC410949#1    (100)   AVDAASSWDEEGVLTPPPSRPRQKVRDERMEQQAGAGCGNELALDEKTAA
      H.vulgare_TC162609#1     (1)   --------MRRSRSSR-REQQRVGRNWQR-----RKTQRPRPWVRGR--K
   O.sativa_Os01g0741900#1    (92)   SSTTTTTGAKRGFIDT-VEDKTEGYNDQK--QQARAGCGKELAVEEM--I
   OsIBCD003255_319_AUX-IAA   (92)   SSTTTTTGAKRGFIDT-VEDKTEGYNDQK--QQARAGCGKELAVEEM--I
     T.aestivum_TC289502#1    (91)   TITTTTTGAKRGFLDT-VEAKAQGYEKEQKQQARAAACGKELAVEEN--T
     T.aestivum_TC297212#1    (91)   TTTTTTTGAKRGFLDT-VEAKAQGYEKEQ---ARAATCGKELAMEEN--T
                Consensus    (101)   T T  TTGAKRGFLDT  E    G    R  Q   AGCGKELAVEE

                                     151                                             200
                     corn      (1)   -MAGETKKGCCCPPSSSHDSDAGPAV---------HRGDVLPVVGWPPVR
   O.sativa_Os05g0523300#1   (136)   -MAGERKKGCCPSPPCSAAAHSSNPQ---------GRGAIPPVVGWPPIR
     T.aestivum_TC350242#1     (1)   --------------------------------------------------
 corn_EU965307__IAA6__ACG37425 (149) AAASERQKGSCCPPTPQHAPPAATVHSGAHVLQLGRR-PSAPVVGWPPVR
        Z.mays_TC410949#1    (150)   AAASERQKGSCCPPTPQHAPPAATVHSGAHVLQLGRR-PSAPVVGWPPVR
      H.vulgare_TC162609#1    (35)   AAVPHRRR----MPLLLLHLRATSATDR----RRGEEGLAVPVVGWPPIR
   O.sativa_Os01g0741900#1   (137)   AAVSERKKGCCPPPPPPHGAPATPAR---NRPQTQGRGAAAPVVGWPPIR
   OsIBCD003255_319_AUX-IAA  (137)   AAVSERKKGCCPPPPPPHGAPATPAR---NRPQTQGR-------------
     T.aestivum_TC289502#1   (138)   ATVGERKKGCCPPPPPPHAPPATPARNIGNRPQARGRGAAAPVVGWPPIR
     T.aestivum_TC297212#1   (135)   EAVGERKKGCCPPPPPAHAPPATPARNIGNRPQARGRGAAAPVVGWPPIR
                Consensus    (151)   AAV ERKKGCCPPPPP HA PATPA       Q  GRG AAPVVGWPPIR

                                     201                                             250
                     corn     (41)   SFRRNLANASSSKQSLEQQQQNDDEASCDKAKQTCKRSPLIKINMDGIPI
   O.sativa_Os05g0523300#1   (176)   SFRRNLTNGSSFKQ----SPERQNDEADDKAKPICKKRPLVKINMDGIPI
     T.aestivum_TC350242#1     (1)   -MELHLSNN---------PLSDKNVEATDRAKPVCKKKPLIKINMDGIPI
 corn_EU965307__IAA6__ACG37425 (198) SFRRNLAHHHHGSS-SKQPTEPQNSEASRKEKPACKKNPLVKINMDGIPI
        Z.mays_TC410949#1    (199)   SFRRNLAHHHHGSS-SKQPTEPQNSEASRKEKPACKKNPLVKINMDGIPI
      H.vulgare_TC162609#1    (77)   SFRRNLASTSA----SKQVHEPQIAEADAKAALNCKKSPLVKINMDGIPI
   O.sativa_Os01g0741900#1   (184)   SFRRNLASSSS----SKHSPEPQNDNANAKVTLTCKKNPLVKINMDGIPI
   OsIBCD003255_319_AUX-IAA  (171)   --R-NLASSSS----SKHSPEPQNDNANAKVTLTCKKNPLVKINMDGIPI
     T.aestivum_TC289502#1   (188)   SFRRNLASTSA----SKQPPEPQIGEADAKAVLDCKKSPLVKINMDGIPI
     T.aestivum_TC297212#1   (185)   SFRRNLASTSA----SKQPPEPQIGEANAKAVLDCKKSPLVKINMDGIPI
                Consensus    (201)   SFRRNLASTSS    SKQPPEPQN EA AKAKL CKK PLVKINMDGIPI
```

# FIGURE 1

```
                                    251                                                300
                        corn  (91)  GRKINLSAYDSYQKLSSAVQDLFCGFLDAQKDESRG----RGAEEKMFSG
      O.sativa_Os05g0523300#1 (222) GRKVDLQIYDSYQKLSSAVEELFRGFLEAQKDLSCAESGEQGAEDKIFSG
         T.aestivum_TC350242#1 (41) GRKVDLASCDNYHKLSSAVEELFRGFLEAQQDLSCDESGVRGAEEKLFSG
   corn_EU965307__IAA6__ACG37425 (247) GRKVDLAAYDSYERLSLGVKELFHGFLQAQKDMS------P-TAGKIFSQ
            Z.mays_TC410949#1 (248) GRKVDLAAYDSYERLSLGVKELFHGFLQAQKDMS------P-TAGKIFSQ
          H.vulgare_TC162609#1 (123) GRKVDLAACDSYERLSLAVKDLFHGFLEVQRDTPRAERAQQGADEKIFSQ
      O.sativa_Os01g0741900#1 (230) GRKIDLAAYNSYDGLSSAVKQLFHGFLQAQKDQTNAQIAQQGADDKIFYQ
     OsIBCD003255_319_AUX-IAA (214) GRKIDLAAYNSYDGLSSAVKQLFHGFLQAQKDQTNAQIAQQGADDKIFYQ
        T.aestivum_TC289502#1 (234) GRKVDLAACDSYGRLSLAVKDLFHGFLQVQRDPSKVDRTQQGADEKIFSQ
        T.aestivum_TC297212#1 (231) GRKVDLAACDSYERLSLAVKDLFHGFLQVQRDPSKVERTQQGADENIFSR
                    Consensus (251) GRKVDLAAYDSYERLSSAVKDLFHGFLQAQKD S AE AQQGADEKIFSQ

                                    301                                                350
                        corn (137) LLDGTGEYTLVCEDSEGGRTLVGHLPWNVFVSTAKRLRVMESSELPHGLI
      O.sativa_Os05g0523300#1 (272) LLDGTGVYTLVYEDNDGDRMLAGDIPWKVFVSTVKRLRVMRRSELPHDMI
         T.aestivum_TC350242#1 (91) LLDGTGEYALVYEDDEGDRMLVGDIPWSVFVSTAKRLRVMRRSELPQRMT
   corn_EU965307__IAA6__ACG37425 (290) LLDGSGEYTLVYEDNEGDRMLVGDVPWNVFVSTAKRLRVLRSSELAKGLV
            Z.mays_TC410949#1 (291) LLDGSGEYTLVYEDNEGDRMLVGDVPWNVFVSTAKRLRVLRSSELAKGLV
          H.vulgare_TC162609#1 (173) LLDGSGEYTLVYEDNEGDRMLVGDVPWNVFVSTAKRLRVLRSSELSHGLA
      O.sativa_Os01g0741900#1 (280) LLDGSGEYTLVYEDSEGDRMLVGDVPWKVFVSTAKRLRVLRSSELSHTLI
     OsIBCD003255_319_AUX-IAA (264) LLDGSGEYTLVYEDSEGDRMLVGDVPWKVFVSTAKRLRVLRSSELSHTLI
        T.aestivum_TC289502#1 (284) LLDGSGEYTLVYEDSEGDRMLVGDVPWNVFVSTAKRLRVLRSSELSHDLI
        T.aestivum_TC297212#1 (281) LLDGSGEYTLVYEDSEGDRMLVGDVPWNVFVSTAKRLRVLRSSELSHGLI
                    Consensus (301) LLDGSGEYTLVYEDSEGDRMLVGDVPWNVFVSTAKRLRVLRSSELSHGLI

                                    351             369
                        corn (187) KTASERADD----------
      O.sativa_Os05g0523300#1 (322) GADPVK-------------
         T.aestivum_TC350242#1 (141) GANSTKVADC---------
   corn_EU965307__IAA6__ACG37425 (340) GTRPPQIAVAPRRGPPDC-
            Z.mays_TC410949#1 (341) GTRPPQIAVAPRRGPPDC-
          H.vulgare_TC162609#1 (223) GVTLERAANC---------
      O.sativa_Os01g0741900#1 (330) GATARV-------------
     OsIBCD003255_319_AUX-IAA (314) GATARV-------------
        T.aestivum_TC289502#1 (334) GATPERAANG---------
        T.aestivum_TC297212#1 (331) GATPERTAHG---------
                    Consensus (351) GATP R A
```

**FIGURE 1 (continued)**

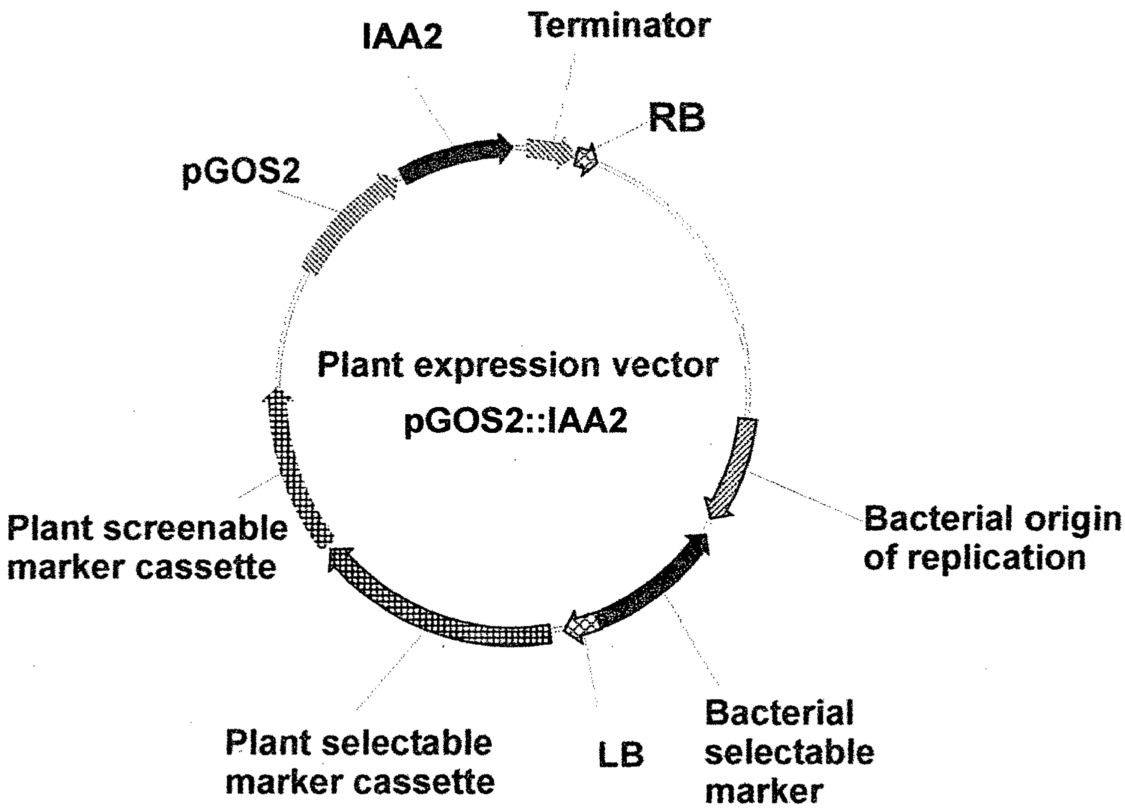
IAA2
Terminator
RB
pGOS2
Plant expression vector
pGOS2::IAA2
Bacterial origin of replication
Plant screenable marker cassette
Plant selectable marker cassette
LB
Bacterial selectable marker

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2004123343 A
- WO 03008540 A
- WO 2007117693 A
- WO 2009016232 A
- US 5811238 A
- US 6395547 A
- WO 2004070039 A
- WO 2004065596 A
- US 4962028 A
- WO 0114572 A
- WO 9514098 A
- WO 9412015 A
- US 5401836 A
- US 20050044585 A
- EP 99106056 A
- US 5565350 A
- WO 0015815 A
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- EP 1198985 A1
- US 5164310 A
- US 5159135 A


### Non-patent literature cited in the description


- **TITTONELL et al.** *Agric Ecosys & Environ,* 2005, vol. 105, 213
- **FASOULA ; TOLLENAAR.** *Maydica,* 2005, vol. 50, 39
- **STEEGE et al.** *Plant Physiology,* 2005, vol. 139, 1078
- **HITTALMANI et al.** *Theoretical Applied Genetics,* 2003, vol. 107, 679
- **REBETZKE et al.** *Crop Science,* 2002, vol. 42, 739
- **GARDENER et al.** Physiology of Crop Plants. Iowa State University Press, 1985, 68-73
- **WANG et al.** *Planta,* 2003, vol. 218, 1-14
- **REED.** *Trends in Plant Science,* 2001, vol. 6, 420-425
- **JAIN et al.** *Funct Integr Genomics,* January 2006, vol. 6 (1), 47-59
- **DE PATER et al.** *The Plant Journal,* 1992, vol. 2 (6), 837-844
- **CREIGHTON.** Proteins. 1984
- **TERPE.** *Appl. Microbiol. Biotechnol.,* 2003, vol. 60, 523-533
- **SCHULTZ et al.** *Proc. Natl. Acad. Sci. USA,* 1998, vol. 95, 5857-5864
- **LETUNIC et al.** *Nucleic Acids Res,* 2002, vol. 30, 242-244
- **MULDER et al.** *Nucl. Acids. Res.,* 2003, vol. 31, 315-318
- A generalized profile syntax for biomolecular sequences motifs and its function in automatic sequence interpretation. **BUCHER ; BAIROCH.** ISMB-94; Proceedings 2nd International Conference on Intelligent Systems for Molecular Biology. AAAI Press, 1994, 53-61
- **HULO et al.** *Nucl. Acids. Res.,* 2004, vol. 32, D134-D137
- **BATEMAN et al.** *Nucleic Acids Research,* 2002, vol. 30 (1), 276-280
- **GASTEIGER et al.** ExPASy: the proteomics server for in-depth protein knowledge and analysis. *Nucleic Acids Res.,* 2003, vol. 31, 3784-3788
- **NEEDLEMAN ; WUNSCH.** *J Mol Biol,* 1970, vol. 48 (X), 443-453
- **ALTSCHUL et al.** *J Mol Biol,* 1990, vol. 215, 403-10
- **CAMPANELLA et al.** *BMC Bioinformatics,* 10 July 2003, vol. 4, 29
- **SMITH TF ; WATERMAN MS.** *J. Mol. Biol,* 1981, vol. 147 (1), 195-7
- **MEINKOTH ; WAHL.** *Anal. Biochem.,* 1984, vol. 138, 267-284
- **SAMBROOK et al.** Molecular Cloning: a laboratory manual. Cold Spring Harbor Laboratory Press, 2001
- Current Protocols in Molecular Biology. John Wiley & Sons, 1989

- **FOISSAC ; SCHIEX.** *BMC Bioinformatics,* 2005, vol. 6, 25
- **CASTLE et al.** *Science,* 2004, vol. 304 (5674), 1151-4
- **HEID et al.** *Genome Methods,* 1996, vol. 6, 986-994
- **MCELROY et al.** *Plant Cell,* 1990, vol. 2, 163-171
- **ODELL et al.** *Nature,* 1985, vol. 313, 810-812
- **NILSSON et al.** *Physiol. Plant.,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet.,* 1992, vol. 231, 276-285
- **WU et al.** *Plant Mol. Biol.,* 1988, vol. 11, 641-649
- **AN et al.** *Plant J.,* 1996, vol. 10 (1), 107-121
- **SANGER et al.** *Plant. Mol. Biol.,* 1990, vol. 14, 433-443
- **LEISNER.** *Proc Natl Acad Sci USA,* 1988, vol. 85 (5), 2553
- **JAIN et al.** *Crop Science,* 1999, vol. 39 (6), 1696
- **SHAW et al.** *Nucleic Acids Res.,* 1984, vol. 12 (20), 7831-7846
- **GATZ.** *Annu. Rev. Plant Physiol. Plant Mol. Biol.,* 1997, vol. 48, 89-108
- *Plant Mol Biol.,* January 1995, vol. 27 (2), 237-48
- **MUDGE et al.** *Plant J.,* 2002, vol. 31, 341
- **NITZ et al.** *Plant Sci,* 2001, vol. 161 (2), 337-346
- **TINGEY et al.** *EMBO J.,* 1987, vol. 6, 1
- **VAN DER ZAAL et al.** *Plant Mol. Biol.,* 1991, vol. 16, 983
- **OPPENHEIMER et al.** *Gene,* 1988, vol. 63, 87
- **CONKLING et al.** *Plant Physiol.,* 1990, vol. 93, 1203
- **SUZUKI et al.** *Plant Mol. Biol.,* 1993, vol. 21, 109-119
- **BAUMBERGER et al.** *Genes & Dev.,* 2001, vol. 15, 1128
- **LAUTER et al.** *PNAS,* 1996, vol. 3, 8139
- **LIU et al.** *Plant Mol. Biol.,* 1991, vol. 153, 386-395
- **DOWNEY et al.** *J. Biol. Chem.,* 2000, vol. 275, 39420
- **W SONG.** *PhD Thesis, North Carolina State University,* 1997
- **WANG et al.** *Plant Sci.,* 2002, vol. 163, 273
- **DIENER et al.** *Plant Cell,* 2001, vol. 13, 1625
- **QUESADA et al.** *Plant Mol. Biol.,* 1997, vol. 34, 265
- **QING QU ; TAKAIWA.** *Plant Biotechnol. J.,* 2004, vol. 2, 113-125
- **SIMON et al.** *Plant Mol. Biol.,* 1985, vol. 5, 191
- **SCOFIELD et al.** *J. Biol. Chem.,* 1987, vol. 262, 12202
- **BASZCZYNSKI et al.** *Plant Mol. Biol.,* 1990, vol. 14, 633
- **PEARSON et al.** *Plant Mol. Biol.,* 1992, vol. 18, 235-245
- **ELLIS et al.** *Plant Mol. Biol.,* 1988, vol. 10, 203-214
- **TAKAIWA et al.** *Mol. Gen. Genet.,* 1986, vol. 208, 15-22
- **TAKAIWA et al.** *FEBS Letts.,* vol. 221, 43-47
- **MATZKE et al.** *Plant Mol Biol,* 1990, vol. 14 (3), 323-32
- **STALBERG et al.** *Planta,* 1996, vol. 199, 515-519
- *Mol Gen Genet,* 1989, vol. 216, 81-90
- *NAR,* 1989, vol. 17, 461-2
- **ALBANI et al.** *Plant Cell,* 1997, vol. 9, 171-184
- *EMBO J.,* 1984, vol. 3, 1409-15
- **DIAZ et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **MENA et al.** *The Plant Journal,* 1998, vol. 116 (1), 53-62
- **VICENTE-CARBAJOSA et al.** *Plant J.,* 1998, vol. 13, 629-640
- **WU et al.** *Plant Cell Physiology,* 1998, vol. 39 (8), 885-889
- **SATO et al.** *Proc. Natl. Acad. Sci. USA,* 1996, vol. 93, 8117-8122
- **NAKASE et al.** *Plant Mol. Biol.,* 1997, vol. 33, 513-522
- *Trans Res,* 1997, vol. 6, 157-68
- *Plant J,* 1997, vol. 12, 235-46
- **DEROSE et al.** *Plant Mol. Biol,* 1996, vol. 32, 1029-35
- **POSTMA-HAARSMA et al.** *Plant Mol. Biol.,* 1999, vol. 39, 257-71
- **WU et al.** *J. Biochem.,* 1998, vol. 123, 386
- **CUMMINS et al.** *Plant Mol. Biol.,* 1992, vol. 19, 873-876
- **LANAHAN et al.** *Plant Cell,* 1992, vol. 4, 203-211
- **SKRIVER et al.** *Proc Natl Acad Sci USA,* 1991, vol. 88, 7266-7270
- **CEJUDO et al.** *Plant Mol Biol,* 1992, vol. 20, 849-856
- **KALLA et al.** *Plant J.,* 1994, vol. 6, 849-60
- **LEAH et al.** *Plant J.,* 1994, vol. 4, 579-89
- **SELINGER et al.** *Genetics,* 1998, vol. 149, 1125-38
- **TAKAIWA et al.** *Mol Gen Genet,* 1986, vol. 208, 15-22

- **TAKAIWA et al.** *FEBS Letts,* 1987, vol. 221, 43-47
- **COLOT et al.** *Mol Gen Genet,* 1989, vol. 216, 81-90
- **ANDERSON et al.** *NAR,* 1989, vol. 17, 461-2
- **ALBANI et al.** *Plant Cell,* vol. 9, 171-184
- **RAFALSKI et al.** *EMBO,* 1984, vol. 3, 1409-15
- **CHO et al.** *Theor Appl Genet,* 1999, vol. 98, 1253-62
- **MULLER et al.** *Plant J,* 1993, vol. 4, 343-55
- **SORENSON et al.** *Mol Gen Genet,* 1996, vol. 250, 750-60
- **MENA et al.** *Plant J,* 1998, vol. 116 (1), 53-62
- **ONATE et al.** *J Biol Chem,* 1999, vol. 274 (14), 9175-82
- **VICENTE-CARBAJOSA et al.** *Plant J,* 1998, vol. 13, 629-640
- **WU et al.** *Plant Cell Physiol,* 1998, vol. 39 (8), 885-889
- **WU et al.** *Plant Cell Physiol,* 1998, vol. 39, 885-889
- **NAKASE et al.** *Plant Molec Biol,* 1997, vol. 33, 513-522
- **RUSSELL et al.** *Trans Res,* 1997, vol. 6, 157-68
- **OPSAHL-FERSTAD et al.** *Plant J,* 1997, vol. 12, 235-46
- **DEROSE et al.** *Plant Mol Biol,* 1996, vol. 32, 1029-35
- **WAGNER ; KOHORN.** *Plant Cell,* 2001, vol. 13 (2), 303-318
- **TRIBBLE et al.** *J. Biol. Chem.,* 2000, vol. 275, 22255-22267
- **VELMURUGAN et al.** *J. Cell Biol.,* 2000, vol. 149, 553-566
- **BUCHMAN ; BERG.** *Mol. Cell biol.,* 1988, vol. 8, 4395-4405
- **CALLIS et al.** *Genes Dev,* 1987, vol. 1, 1183-1200
- The Maize Handbook. Springer, 1994
- **GAULTIER et al.** *Nucl Ac Res,* 1987, vol. 15, 6625-6641
- **INOUE et al.** *Nucl Ac Res,* 1987, vol. 15, 6131-6148
- **INOUE et al.** *FEBS Lett.,* 1987, vol. 215, 327-330
- **HASELHOFF ; GERLACH.** *Nature,* vol. 334, 585-591
- **BARTEL ; SZOSTAK.** *Science,* 1993, vol. 261, 1411-1418
- **ANGELL ; BAULCOMBE.** *Plant J,* 1999, vol. 20 (3), 357-62
- **HELENE, C.** *Anticancer Drug Res.,* 1991, vol. 6, 569-84
- **HELENE et al.** *Ann. N.Y. Acad. Sci.,* 1992, vol. 660, 27-36
- **MAHER, L.J.** *Bioassays,* 1992, vol. 14, 807-15
- **SCHWAB et al.** *Dev. Cell,* 2005, vol. 8, 517-527
- **SCHWAB et al.** *Plant Cell,* 2006, vol. 18, 1121-1133
- **KRENS, F.A. et al.** *Nature,* 1982, vol. 296, 72-74
- **NEGRUTIU I et al.** *Plant Mol Biol,* 1987, vol. 8, 363-373
- **SHILLITO R.D. et al.** *Bio/Technol,* 1985, vol. 3, 1099-1102
- **CROSSWAY A et al.** *Mol. Gen Genet,* 1986, vol. 202, 179-185
- **KLEIN TM et al.** *Nature,* 1987, vol. 327, 70
- **CLOUGH ; BENT.** *Plant J.,* 1998, vol. 16, 735-743
- **ALDEMITA ; HODGES.** *Planta,* 1996, vol. 199, 612-617
- **CHAN et al.** *Plant Mol Biol,* 1993, vol. 22 (3), 491-506
- **HIEI et al.** *Plant J,* 1994, vol. 6 (2), 271-282
- **ISHIDA et al.** *Nat. Biotechnol,* 1996, vol. 14 (6), 745-50
- **FRAME et al.** *Plant Physiol,* 2002, vol. 129 (1), 13-22
- **B. JENES et al.** Techniques for Gene Transfer, in: Transgenic Plants, Vol. 1, Engineering and Utilization. Academic Press, 1993, 128-143
- *Potrykus Annu. Rev. Plant Physiol. Plant Molec. Biol.,* 1991, vol. 42, 205-225
- **BEVAN et al.** *Nucl. Acids Res.,* 1984, vol. 12, 8711
- **HÖFGEN ; WILLMITZER.** *Nucl. Acid Res.,* 1988, vol. 16, 9877
- Vectors for Gene Transfer in Higher Plants. **F.F. WHITE.** Transgenic Plants, Vol. 1, Engineering and Utilization. Academic Press, 1993, vol. 1, 15-38
- **FELDMAN, KA ; MARKS MD.** *Mol Gen Genet,* 1987, vol. 208, 274-289
- **FELDMANN K.** Methods in Arabidopsis Research. Word Scientific, 274-289
- **CHANG.** *Plant J.,* 1994, vol. 5, 551-558
- **KATAVIC.** *Mol Gen Genet,* 1994, vol. 245, 363-370
- **BECHTHOLD, N.** *C R Acad Sci Paris Life Sci,* 1993, vol. 316, 1194-1199
- **CLOUGH, SJ ; BENT AF.** *The Plant J,* 1998, vol. 16, 735-743
- **KLAUS et al.** *Nature Biotechnology,* 2004, vol. 22 (2), 225-229
- **BOCK.** Transgenic plastids in basic research and plant biotechnology. *J Mol Biol.,* 21 September 2001, vol. 312 (3), 425-38
- **MALIGA, P.** Progress towards commercialization of plastid transformation technology. *Trends Biotechnol.,* 2003, vol. 21, 20-28
- **HAYASHI et al.** *Science,* 1992, 1350-1353

- **REDEI GP ; KONCZ C.** In Methods in Arabidopsis Research. World Scientific Publishing Co, 1992, 16-82
- **FELDMANN et al.** Arabidopsis. Cold Spring Harbor Laboratory Press, 1994, 137-172
- **LIGHTNER J ; CASPAR T.** Methods on Molecular Biology. Humana Press, 1998, vol. 82, 91-104
- **MCCALLUM et al.** *Nat Biotechnol,* 2000, vol. 18, 455-457
- **STEMPLE.** *Nat Rev Genet,* 2004, vol. 5 (2), 145-50
- **OFFRINGA et al.** *EMBO J,* 1990, vol. 9 (10), 3077-84
- **TERADA et al.** *Nat Biotech,* 2002, vol. 20 (10), 1030-4
- **LIDA ; TERADA.** *Curr Opin Biotech,* 2004, vol. 15 (2), 132-8
- **MILLER et al.** *Nature Biotechnol.,* 2007, vol. 25, 778-785
- **RABBANI et al.** *Plant Physiol,* 2003, vol. 133, 1755-1767
- **SAMBROOK J ; FRITSCH EF ; MANIATIS T.** Molecular Cloning. A Laboratory Manual, 1989
- **LANDER et al.** *Genomics,* 1987, vol. 1, 174-181
- **BOTSTEIN et al.** *Am. J. Hum. Genet.,* 1980, vol. 32, 314-331
- **BERNATZKY ; TANKSLEY.** *Plant Mol. Biol. Reporter,* 1986, vol. 4, 37-41
- **HOHEISEL et al.** Non-mammalian Genomic Analysis: A Practical Guide. Academic press, 1996, 319-346
- **TRASK.** *Trends Genet,* 1991, vol. 7, 149-154
- **LAAN et al.** *Genome Res,* 1995, vol. 5, 13-20
- **KAZAZIAN.** *J. Lab. Clin. Med,* 1989, vol. 11, 95-96
- **SHEFFIELD et al.** *Genomics,* 1993, vol. 16, 325-332
- **LANDEGREN et al.** *Science,* 1988, vol. 241, 1077-1080
- **SOKOLOV.** *Nucleic Acid Res.,* 1990, vol. 18, 3671
- **WALTER et al.** *Nat. Genet.,* 1997, vol. 7, 22-28
- **DEAR ; COOK.** *Nucleic Acid Res.,* vol. 17, 6795-6807
- A generalized profile syntax for biomolecular sequences motifs and its function in automatic sequence interpretation. **BUCHER ; BAIROCH.** In) ISMB-94; Proceedings 2nd International Conference on Intelligent Systems for Molecular Biology. AAAI Press, 1994, 53-61
- **GASTEIGER et al.** *Nucleic Acids Res.,* 2003, vol. 31, 3784-3788
- **NEEDLEMAN ; WUNSCH.** *J Mol Biol,* 1970, vol. 48, 443-453
- **FUKAKI et al.** *Plant J.,* 2005, vol. 44, 382-395
- Current Protocols in Molecular Biology
- **FRINK et al.** *Proc Natl Acad Sci USA,* 1999, vol. 96 (4), 1175-1180
- **SAMBROOK J ; FRITSCH EF ; MANIATIS T.** Molecular Cloning, A Laboratory Manual. 1989
- **LAAN et al.** *Genome Res.,* 1995, vol. 5, 13-20
- **DEAR ; COOK.** *Nucleic Acid Res.,* 1989, vol. 17, 6795-6807
- **SAMBROOK.** Molecular Cloning: a laboratory manual. Cold Spring Harbor Laboratory Press, 2001
- **AUSUBEL et al.** Current Protocols in Molecular Biology, Current Protocols. 1994, vol. 1, 2
- **R.D.D. CROY.** Plant Molecular Biology Labfax. BIOS Scientific Publications Ltd, 1993
- **ALTSCHUL et al.** *J. Mol. Biol.,* 1990, vol. 215, 403-410
- **ALTSCHUL et al.** *Nucleic Acids Res.,* 1997, vol. 25, 3389-3402
- **THOMPSON et al.** *Nucleic Acids Res,* 1997, vol. 25, 4876-4882
- **CHENNA et al.** *Nucleic Acids Res,* 2003, vol. 31, 3497-3500
- **CAMPANELLA JJ ; BITINCKA L ; SMALLEY J.** MatGAT: an application that generates similarity/identity matrices using protein or DNA sequences. *BMC Bioinformatics,* 2003, vol. 4, 29
- **PARK ; KANEHISA.** *Bioinformatics,* 2003, vol. 19, 1656-1663
- **ISHIDA et al.** *Nature Biotech,* 1996, vol. 14 (6), 745-50
- **BABIC et al.** *Plant Cell Rep,* 1998, vol. 17, 183-188
- **MCKERSIE et al.** *Plant Physiol,* 1999, vol. 119, 839-847
- **BROWN DCW ; A ATANASSOV.** *Plant Cell Tissue Organ Culture,* 1985, vol. 4, 111-112
- **WALKER et al.** *Am J Bot,* 1978, vol. 65, 654-659
- **GAMBORG et al.** *Exp. Cell Res.,* 1968, vol. 50, 151-158